(19) Europäisches Patentamt
European Patent Office
Office européen des brevets

(11) EP 4 442 246 A1

(12) EUROPEAN PATENT APPLICATION
published in accordance with Art. 153(4) EPC

(43) Date of publication:
09.10.2024 Bulletin 2024/41

(21) Application number: 22901242.2

(22) Date of filing: 28.11.2022

(51) International Patent Classification (IPC):
*A61K 8/89* (2006.01) *A61K 8/894* (2006.01)
*A61Q 3/02* (2006.01) *A61Q 5/10* (2006.01)

(52) Cooperative Patent Classification (CPC):
**A61K 8/31; A61K 8/37; A61K 8/58; A61K 8/81; A61K 8/89; A61K 8/891; A61K 8/894; A61K 8/898; A61Q 3/02; A61Q 5/00; A61Q 5/10**

(86) International application number:
PCT/JP2022/043778

(87) International publication number:
WO 2023/100810 (08.06.2023 Gazette 2023/23)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 30.11.2021 JP 2021194582

(71) Applicant: **Kao Corporation**
**Tokyo 103-8210 (JP)**

(72) Inventor: **SHIMMEN, Manami**
**Tokyo 131-8501 (JP)**

(74) Representative: **Hoffmann Eitle**
**Patent- und Rechtsanwälte PartmbB**
**Arabellastraße 30**
**81925 München (DE)**

# (54) METHOD FOR TREATING KERATIN SUBSTANCE OR FIBER FOR HEAD DECORATION PRODUCTS

(57) Provided is a method for treating a keratin substance or a fiber for a head decoration product, the method including, in the following order, a step (I) of applying a first agent to the keratin substance or the fiber for a head decoration product to form a film, and a step (II) of applying a second agent to the keratin substance or the fiber for a head decoration product on which the film has been formed to remove the film, in which the first agent contains a component (A): a silicone film-forming agent that is solid at 25°C, and a component (B): an organopolysiloxane other than the component (A), which has a cationic group, and the second agent contains a component (C): an oil agent.

EP 4 442 246 A1

## Description

### Field of the Invention

The present invention relates to a method for treating a keratin substance or a fiber for a head decoration product.

### Background of the Invention

Keratin substances are sometimes colored for the purpose of improving fashionability, such as dyeing of hair with a hair dye or dyeing of nails with a manicure. In addition, there is an increasing need to freely change hair color and nail color at a desired timing, for example, to change the color of hair or nails casually, or to color the hair or nails during a vacation and then restore the color after the vacation.

On the other hand, the coloring effect of a keratin substance or a fiber for a head decoration product such as a wig may be lost by rubbing due to daily living activities or by washing once. Therefore, a technique has been studied in which the coloring durability when the keratin substance or the fiber for a head decoration product is colored is good and the coloring can be easily restored when it is desired to turn off the coloring.

For example, JP 2006-63059 A (PTL1) discloses a colored hair dye that can release colored hair dyeing with a neutralizing agent, which is characterized in that a neutralizing reaction-type resin that exhibits water resistance when not neutralized and becomes clearly water-soluble after neutralization has a washing resistance such that the film after drying in an unneutralized state cannot be easily washed away by an acidic or neutral hair detergent is used as an adhesive resin and is mixed and dissolved with a non-aqueous solvent together with a colorant, and a colored hair dyeing method and a colored hair dyeing release method using the same. It is described that, according to the colored hair dye, after enjoying "fashionable dyeing" using a colored hair dye of a desired color for a certain period of time, the effect can be released instantly at any time, and the hair can be returned to the original hair color and hair quality.

### Summary of the Invention

The present invention relates to the following.

[1] A method for treating a keratin substance or a fiber for a head decoration product, the method including, in the following order,

a step (I) of applying a first agent to the keratin substance or the fiber for a head decoration product to form a film, and
a step (II) of applying a second agent to the keratin substance or the fiber for a head decoration product on which the film has been formed to remove the film,
wherein the first agent contains a component (A): a silicone film-forming agent that is solid at 25°C, and a component (B): an organopolysiloxane other than the component (A), which has a cationic group, and the second agent contains a component (C): an oil agent.

[2] A kit for treating a keratin substance or a fiber for a head decoration product, including: a first agent containing a component (A): a silicone film-forming agent that is solid at 25°C, and a component (B): an organopolysiloxane other than the component (A), which has a cationic group, and a second agent containing a component (C): an oil agent.

### Detailed Description of the Invention

The durability of the treatment effect of the keratin substance or the fiber for a head decoration product by the treatment agent and the removability of the treatment agent are usually in a trade-off relationship. Even in the technique disclosed in PTL 1, the durability of the colored hair dyeing effect is not sufficient. Further, in practical use, the quick-drying property of the hair after releasing the colored hair dyeing and the feel of the hair after treatment are also important, but PTL 1 does not refer to these.

The present invention relates to a method for treating a keratin substance or a fiber for a head decoration product, in which the durability of the treatment effect of the keratin substance or the fiber for a head decoration product by the treatment agent is high, the feel after the treatment is good, the treatment agent can be easily removed from the keratin substance or the fiber for a head decoration product, and both the quick-drying property and the feel of the keratin substance or the fiber for a head decoration product after removal of the treatment agent are excellent.

The present inventor has found that the above problem can be solved by a method including a step of treating

a keratin substance or a fiber for a head decoration product with a predetermined first agent and a predetermined second agent, and has thus completed the present invention.

According to the present invention, it is possible to provide a treatment method in which the durability of the treatment effect of the keratin substance or the fiber for a head decoration product by the treatment agent is high, the feel after the treatment is good, the treatment agent can be easily removed from the keratin substance or the fiber for a head decoration product, and both the quick-drying property and the feel of the keratin substance or the fiber for a head decoration product after removal of the treatment agent are excellent. For example, when the first agent is a hair dye containing a colorant, the hair dyed with the hair dye has high hair washing resistance, excellent color durability, and good feel after dyeing. In addition, the coloring by the hair dye can be easily restored by treatment with the second agent, and the hair after removal of the coloring is excellent in quick-drying property and feel.

[Definitions]

"Polymer" as used herein means a compound corresponding to a repetition of one or plural units (these units are derived from a compound known as a monomer). This or these units are repeated at least two times preferably at least three times.

"Keratin substance" as used herein includes, for example, the skin, eyebrows, eyelashes, hair, and nails of an animal such as a human being, as well as artificially produced keratin substances (excluding fibers for a head decoration product).

"Hair" as used herein means human head hair, but also includes hair that has been cut from a human head.

"Head decoration product" as used herein means, for example, a hair wig, a wig, weaving, hair extension, a blade hair, a hair accessory, and a doll hair.

"Fiber for a head decoration product" as used herein means a fiber used in the head decoration product.

"Hydrophobic" as used herein means that a solubility in water of a substance is less than 1% by mass at 25°C.

"Film formation" as used herein means that, when applied to a substrate, a solid film is left thereon.

"Volatile" as used herein means a substance having a boiling point of 260°C or lower under normal pressure.

[Method for Treating Keratin Substance or Fiber for Head Decoration Product]

The method for treating a keratin substance or a fiber for a head decoration product of the present invention (hereinafter also referred to as the "method of the present invention") is a method including, in the following order, a step (I) of applying a first agent to the keratin substance or the fiber for a head decoration product to form a film, and a step (II) of applying a second agent to the keratin substance or the fiber for a head decoration product on which the film has been formed to remove the film, wherein the first agent contains a component (A): a silicone film-forming agent that is solid at 25°C, and a component (B): an organopolysiloxane other than the component (A), which has a cationic group, and the second agent contains a component (C): an oil agent.

The present invention has the above-mentioned configuration, and thus the durability of the treatment effect of the keratin substance or the fiber for a head decoration product after treatment with the treatment agent (first agent) is high, the feel is good, and the treatment agent (film formed by the first agent) can be easily removed from the keratin substance or the fiber for a head decoration product by the second agent, and both the quick-drying property and the feel of the keratin substance or the fiber for a head decoration product after removal of the treatment agent (film) are excellent.

In the description herein, "the feel of the keratin substance or the fiber for a head decoration product after treatment with the treatment agent (first agent) is good" means that the keratin substance or the fiber for a head decoration product after treatment with the first agent in the step (I) has a smooth feel, and when the keratin substance is hair, it means that the finger combability of the hair after treatment is good.

Further, "the feel of the keratin substance or the fiber for a head decoration product after removal of the treatment agent is good" means that the keratin substance or the fiber for a head decoration product after removal of the film in the step (II) does not have stickiness.

The above-mentioned effects of the present invention can be evaluated specifically by the methods described in Examples.

The reason why the present invention exhibits the above-mentioned effects is not clear, but is presumed as follows.

In the treatment method of the present invention, the first agent used in the step (I) contains a component (A) and a component (B). The component (A) is a hydrophobic film-forming agent that is solid at 25°C, and a hydrophobic film can be formed by applying the first agent containing the component (A) to a keratin substance or a fiber for a head decoration product. Since the component (A) can maintain the strength and durability of the film, it is considered that the durability of the film formed on the surface of the keratin substance or the fiber for a head decoration product against

friction and washing can be improved by the first agent, and the treatment effect by the first agent can be maintained. It is also considered that the quick-drying property of the keratin substance or the fiber for a head decoration product after the removal of the film by the step (II) can be improved and stickiness can be suppressed. Furthermore, in the case where the first agent contains a colorant such as a pigment, it is considered that the color durability after washing of the keratin substance or the fiber for a head decoration product is further improved due to the effect of retaining the colorant in the film.

On the other hand, it is considered that the component (B) can improve the feel of the keratin substance after treatment with the first agent because it has a cationic group. Furthermore, since the component (B) has high adsorptivity to the keratin substance or the fiber for a head decoration product having a negative charge, it is considered that the component (B) is unevenly distributed on the keratin substance or the fiber for a head decoration product side in the film formed by the first agent, strongly adheres the film to the surface thereof, and enhances the peeling prevention effect of the film.

The second agent used in the step (II) contains a component (C): an oil agent. The oil agent is preferably an oil agent that is liquid at 25°C, is easily compatible with the film formed on the surface of the keratin substance or the fiber for a head decoration product in the step (I), and is considered to be capable of easily peeling, dispersing, and removing the film without impairing the quick-drying property and the feel of the keratin substance or the fiber for a head decoration product after the film is removed.

Therefore, for example, when the first agent is a hair dye containing a colorant, the hair dyed with the hair dye has high hair washing resistance and excellent color durability, and the feel after dyeing is also good. In addition, it is considered that the coloring by the hair dye can be easily restored by treating with the second agent, and the quick-drying property and the feel of the hair after removing the hair dye become good.

It should be noted that the action mechanism of the present invention is not limited to the above.

<Keratin Substance or Fiber for Head Decoration Product>

As the keratin substance to which the method of the present invention is applied, skin, eyebrows, eyelashes, hair, and nails are preferable. Among these, hair is more preferable as the keratin substance from the viewpoint of applying the second agent.

The fiber for a head decoration product to which the method of the present invention is applied may be either a naturally derived fiber or a synthetic fiber, and a naturally derived fiber is preferable. The naturally derived fiber refers to a fiber collected from a natural animal or plant (excluding human hair) or a fiber artificially produced using a protein or a polysaccharide as a raw material. Of these, fibers artificially produced from animal hair or proteins or polysaccharides such as proteins derived from keratin, collagen, casein, soybean, peanut, corn, and silk are preferable, regenerated protein fibers from keratin, collagen, casein, soybean protein, peanut protein, corn protein, and silk protein (for example, silk fibroin) are more preferable, regenerated protein fibers such as regenerated collagen fibers from collagen and regenerated silk fibers from silk fibroin are more preferable, and regenerated collagen fibers are still more preferable.

The regenerated collagen fibers can be produced by a known technique. The composition of the regenerated collagen fibers need not be 100% collagen, and may contain natural polymers, synthetic polymers, and additives for quality improvement. Furthermore, the regenerated collagen fibers may be post-treated.

The regenerated collagen fibers are preferably in the form of filaments. The filament is generally taken out from a bobbin-wound state or a packed state. It is also possible to directly utilize the filament coming out from the drying step in the production process of the regenerated collagen fiber.

Examples of the synthetic fiber include a fiber containing a synthetic resin as a main component. From the viewpoint of ease of production of the synthetic fiber and from the viewpoint of obtaining a texture close to that of hair, the synthetic resin is preferably a thermoplastic resin, and more preferably one or more selected from the group consisting of a polyester resin, a polyamide resin, a polyimide resin, a polyamide-imide resin, a vinyl chloride resin, a polycarbonate resin, a polyphenylene sulfide resin, and a modacrylic resin (a copolymer of acrylonitrile and vinyl chloride). The term "main component" as used herein means a component having a content in the synthetic fiber of preferably 50% by mass or more, more preferably 60% by mass or more, still more preferably 70% by mass or more, even more preferably 80% by mass or more, and even more preferably 90% by mass or more, and 100% by mass or less.

The synthetic fiber may further contain various components such as a flame retardant, a flame retardant aid, a light or heat stabilizer, a fluorescent agent, an antioxidant, an anti-static agent, and an ultraviolet absorber in addition to the synthetic resin within a range not inhibiting the effect of the present invention.

The application object of the method of the present invention is preferably skin, eyebrows, eyelashes, hair, nails, or fibers for head decoration products, more preferably hair or fibers for head decoration products, and even more preferably hair.

<Step (I)>

Step (I) is a step of applying a first agent to a keratin substance or a fiber for a head decoration product to form a film, and the first agent contains a component (A): a silicone film-forming agent that is solid at 25°C, and a component (B): an organopolysiloxane other than the component (A), which has a cationic group. Hereinafter, the components contained in the first agent used in step (I) will be described.

(Component (A): Silicone Film-Forming Agent that is Solid at 25°C)

The first agent contains a silicone film-forming agent that is solid at 25°C as the component (A). By containing the component (A), a hydrophobic film having high durability against friction and washing can be formed when the first agent is applied to a keratin substance or a fiber for a head decoration product. Hereinafter, high durability against friction and washing is also referred to as "durability of the treatment effect of a keratin substance or a fiber for a head decoration product".

As the component (A), a silicone film-forming agent that is solid at 25°C and is usually used in cosmetic materials can be used.

From the viewpoint of film formability and improving the durability of the treatment effect of the keratin substance or the fiber for a head decoration product, the component (A) is preferably one or more selected from the group consisting of the following components (A1) and (A2):

Component (A1): a silicone resin represented by an average formula, $(R^1)_mSiO_{(4-m)/2}$ (in which $R^1$ represents a hydrocarbon group having 1 or more and 12 or less carbon atoms and optionally substituted with fluorine, or a hydroxy group, plural $R^1$'s can be the same as or different from each other, and m is an average number, representing a number of more than 0 and less than 3), which contains one or more units selected from the group consisting of a T unit represented by $R^1SiO_{3/2}$ and a Q unit represented by $SiO_{4/2}$.

Component (A2): a silicone polymer containing a polysiloxane moiety and a moiety formed of a non-silicone organic chain.

Preferably, the component (A1) includes those containing one or more selected from the group consisting of the following component (A1-1) and component (A1-2).

(A1-1) A silicone resin represented by the above-mentioned average formula, containing a T unit represented by $R^1SiO_{3/2}$ and substantially not containing a Q unit represented by $SiO_{4/2}$.

(A1-2) A silicone resin represented by the above-mentioned average formula, and containing a Q unit represented by $SiO_{4/2}$ and an M unit represented by $(R^1)_3SiO_{1/2}$.

Preferably, the component (A2) includes those containing one or more selected from the group consisting of the following components (A2-1) to (A2-4).

(A2-1) An acryl silicone polymer.

(A2-2) A silicone-modified alicyclic structure-containing polymer.

(A2-3) A silicone-modified pullulan.

(A2-4) A polyurea/urethane silicone.

(Component (A1))

The component (A1) is a silicone resin represented by an average formula, $(R^1)_mSiO_{(4-m)/2}$ (in which $R^1$ represents a hydrocarbon group having 1 or more and 12 or less carbon atoms and optionally substituted with fluorine, or a hydroxy group, plural $R^1$'s can be the same as or different from each other, and m is an average number, representing a number of more than 0 and less than 3), which contains one or more units selected from the group consisting of a T unit represented by $R^1SiO_{3/2}$ and a Q unit represented by $SiO_{4/2}$.

The component (A1) is represented by the above-mentioned average formula and contains one or more units selected from the group consisting of the above-mentioned T unit and Q unit, and therefore has a crosslinked structure in the molecule. Having the structure, the silicone resin is considered to be able to form a film having higher durability. The component (A1) does not contain a polyorganosiloxane cured product powder which is infusible and does not have a softening point and which is generally insoluble in an organic solvent.

In the average formula, $R^1$ represents a hydrocarbon group having 1 or more and 12 or less carbon atoms and optionally substituted with fluorine, or a hydroxy group. The number of carbon atoms of the hydrocarbon group is 1 or more, and is preferably 9 or less, more preferably 6 or less, and even more preferably 4 or less, from the viewpoint of film formability in the step (I) and improving the durability of the treatment effect of the keratin substance or the fiber for a head decoration product.

The hydrocarbon group may be any of an aliphatic group or an aromatic group, and examples thereof include an alkyl group, an alkenyl group, an aryl group, and an aralkyl group. The alkyl group and the alkenyl group may be

linear or branched.

Among the above, from the viewpoint of availability and stability, the hydrocarbon group is preferably an alkyl group, an aryl group, or an aralkyl group.

The alkyl group includes a methyl group, an ethyl group, a n-propyl group, an isopropyl group, various butyl groups, various pentyl groups, various hexyl groups, various heptyl groups, various octyl groups, various nonyl group, various decyl groups, various undecyl groups, and various dodecyl groups. The word "various" means a linear or branched hydrocarbon group, and for example, "various butyl groups" include "a n-butyl group, a secbutyl group, an isobutyl group, and a tert-butyl group".

The aryl group includes a phenyl group, a toluyl group, a dimethylphenyl group, and a naphthyl group, and is preferably a phenyl group.

The aralkyl group includes a benzyl group, a phenylethyl group, a phenylpropyl group, and a phenylbutyl group, and is preferably a phenylpropyl group.

In the case where $R^1$ is substituted with fluorine, at least one hydrogen atom of the hydrocarbon group may be substituted with a fluorine atom.

$R^1$ is, from the viewpoint of film formability and improving the durability of the treatment effect of the keratin substance or the fiber for a head decoration product, preferably an optionally fluorine-substituted, alkyl group having 1 or more and 12 or less carbon atoms, aryl group having 6 or more and 12 or less carbon atoms or aralkyl group having 7 or more and 12 or less carbon atoms, more preferably an optionally fluorine-substituted, alkyl group having 1 or more and 8 or less carbon atoms or phenyl group, even more preferably an optionally fluorine-substituted, alkyl group having 1 or more and 6 or less carbon atoms or phenyl group. The fluorine-substituted alkyl group is preferably a group represented by $CF_3$-R- in which R represents an alkylene group having 2 or more and 7 or less carbon atoms, preferably 2 or more and 5 or less carbon atoms.

$R^1$ is even more preferably a trifluoropropyl group, an alkyl group having 1 or more and 4 or less carbon atoms, or a phenyl group, even more preferably a trifluoropropyl group, a methyl group, an ethyl group, a n-propyl group, an isopropyl group or a n-butyl group, even more preferably a trifluoropropyl group, a methyl group or a n-propyl group, and even more preferably a methyl group.

The component (A1) may contain one or more units selected from the group consisting of a T unit represented by $R^1SiO_{3/2}$ and a Q unit represented by $SiO_{4/2}$ and, from the viewpoint of film formability and improving the durability of the treatment effect of the keratin substance or the fiber for a head decoration product, preferably further contains one or more units selected from the group consisting of an M unit represented by $(R^1)_3SiO_{1/2}$ and a D unit represented by $(R^1)_2SiO_{2/2}$. $R^1$ is the same as above.

From the viewpoint of film formability and improving the durability of the treatment effect of the keratin substance or the fiber for a head decoration product, the component (A1) is preferably one or more selected from the group consisting of a silicone resin (A1-1) represented by the above-mentioned average formula, containing a T unit represented by $R^1SiO_{3/2}$ and substantially not containing a Q unit represented by $SiO_{4/2}$, and a silicone resin (A1-2) represented by the above-mentioned average formula, and containing a Q unit represented by $SiO_{4/2}$ and an M unit represented by $(R^1)_3SiO_{1/2}$.

[Silicone Resin (A1-1)]

The silicone resin (A1-1) represented by the above-mentioned average formula, containing a T unit represented by $R^1SiO_{3/2}$ and substantially not containing a Q unit represented by $SiO_{4/2}$ (hereinafter also referred to as "component (A1-1)") is a silicone resin containing a T unit and optionally containing an M unit and a D unit, and is preferably a silicone resin represented by $[R^1SiO_{3/2}]_{a[}(R^1)_3SiO_{1/2}]_b$ in which a and b each are an average repeating unit number, and a > 0 and b ≥ 0, and containing a T unit and optionally containing an M unit. The wording "substantially not containing XX" means the constituent ratio of XX in the silicone resin is less than 1 mol%.

$R^1$ is the same as above, and is preferably an alkyl group having 1 or more and 4 or less carbon atoms or a phenyl group, more preferably a methyl group, an ethyl group, a n-propyl group or an isopropyl group, and even more preferably a methyl group, a n-propyl group, or an isopropyl group.

The component (A1-1) includes polysilsesquioxanes such as polymethylsilsesquioxane, polypropylsilsesquioxane, polyphenylsilsesquioxane, polymethylphenylsilsesquioxane, and fluorine-modified alkyldimethylpolysilsesquioxanes, and among these, one or more can be used. Fluorine-modified alkyldimethylpolysilsesquioxanes include, as INCI nomenclature, (trifluoropropyldimethylsiloxy/trimethylsiloxy)silsesquioxane.

Among these, as the component (A1-1), one or more selected from the group consisting of polymethylsilsesquioxane and polypropylsilsesquioxane is preferable, and polymethylsilsesquioxane is more preferable, from the viewpoint of film formability and improving the durability of the treatment effect of the keratin substance or the fiber for a head decoration product.

Commercially available products of the component (A1-1) include SilForm Flexible Resin (polymethylsilsesquioxane), SilForm FR-5 (polydimethylsiloxane solution of (trifluoropropyldimethylsiloxy/trimethylsiloxy)silsesquioxane) (all manufactured by Momentive Performance Materials, Inc.), DOWSIL 680 ID Fluid (isododecane solution of 75% by mass polypropylsilsesquioxane) (manufactured by Dow Toray Co., Ltd.), SR-21 (polyphenylsilsesquioxane), SR-23 (polyphenylsilsesquioxane), SR-33 (polymethylphenylsilsesquioxane) (all manufactured by Konishi Chemical Ind. Co., Ltd.).

[Silicone Resin (A1-2)]

The silicone resin (A1-2) represented by the above-mentioned average formula, and containing a Q unit represented by $SiO_{4/2}$ and an M unit represented by $(R^1)_3SiO_{1/2}$ (hereinafter also referred to as "component (A1-2)") is a silicone resin substantially containing a Q unit and an M unit and optionally containing a D unit or a T unit, and is preferably a silicone resin represented by $[SiO_{4/2}]_c[(R^1)_3SiO_{1/2}]_d$ in which c and d each are an average repeating unit number and $c > 0$ and $d > 0$.

$R^1$ is the same as above, and is preferably an optionallyfluorine-substituted, alkyl group having 1 or more and 6 or less carbon atoms or phenyl group, more preferably a trifluoropropyl group, an alkyl group having 1 or more and 4 or less carbon atoms, or a phenyl group, even more preferably a trifluropropyl group, a methyl group, an ethyl group, a n-propyl group, or an isopropyl group, even more preferably a trifluoropropyl group or a methyl group, and even more preferably a methyl group.

The component (A1-2) includes trimethylsiloxysilicate, phenylpropyldimethylsiloxysilicate, fluorine-modified alkyldimethylsiloxysilicates, and crosspolymers produced by crosslinking these siloxysilicates with dimethiconol, and one or more of these can be used. Fluorine-modified alkyldimethylsiloxysilicates include trifluoroalkyldimethyltrimethylsiloxysilicate, such as trifluoropropyldimethyltrimethylsiloxysilicate of, as INCI nomenclature, trifluoropropyldimethyl/trimethylsiloxysilicate. Crosspolymers produced by crosslinking siloxysilicates with dimethiconol include, as INCI nomenclature, (trimethylsiloxysilicate/dimethiconol) crosspolymer.

Above all, from the viewpoint of film formability and improving the durability of the treatment effect of the keratin substance or the fiber for a head decoration product, the component (A1-2) is preferably one or more selected from the group consisting of trimethylsiloxysilicate, trifluoropropyldimethyltrimethylsiloxysilicate and (trimethylsiloxysilicate/dimethiconol) crosspolymer, more preferably one or more selected from the group consisting of trimethylsiloxysilicate, and trifluoropropyldimethyltrimethylsiloxysilicate, and even more preferably trimethylsiloxysilicate.

Commercially available products of trimethylsiloxysilicate of the component (A1-2) include KF-7312J (50% by mass decamethylcyclopentasiloxane solution), KF-9021 (50% by mass decamethylcyclopentasiloxane solution), X-21-5249(50% by mass decamethylcyclopentasiloxane solution), X-21-5595 (60% by mass isododecane solution), and X-21-5616 (60% by mass isododecane solution) (all manufactured by Shin-Etsu Chemical Co., Ltd.), SS4267 (35% by mass dimethylpolysiloxane solution), SR1000, SS4230 (45% by mass cyclopentasiloxane solution), SS4267 (35% by mass dimethylpolysiloxane solution), and Silsoft 74 (75% by mass isododecane solution) (all manufactured by Momentive Performance Materials, Inc.), BY11-018 (30% by mass cyclopentasiloxane solution), and MQ-1600 Solid Resin (all manufactured by Dow Toray Co., Ltd.), and BELSIL TMS 803 (manufactured by Wacker Asahikasei Silicone Co., Ltd.).

Commercially available products of phenylpropyldimethylsiloxysilicate include SilShine 151 (manufactured by Momentive Performance Materials, Inc.).

Commercially available products of fluorine-modified alkyldimethylsiloxysilicates include, XS66-B8226 (50% by mass cyclopentasiloxane solution), XS66-C1191, and XS66-B8636 (50% by mass dimethicone solution) (all manufactured by Momentive Performance Materials, Inc.), as INCI nomenclature, (trifluoropropyldimethyl/trimethylsiloxysilicate).

Commercially available products of trimethylsiloxysilicate crosspolymer include DOWSIL FC-5001 CM Resin Gum (40% by mass decamethylcyclopentasiloxane solution of (trimethylsiloxysilicate/dimethiconol) crosspolymer), DOWSIL FC-5002 IDD Resin Gum (40% by mass isododecane solution of (trimethylsiloxysilicate/dimethiconol) crosspolymer), and DOWSIL FC-5004 DM (1.5 cSt) Silicone Resin Gum (40% by mass dimethicone solution of (trimethylsiloxysilicate/dimethiconol) crosspolymer) (all manufactured by Dow Toray Co., Ltd.).

In addition, commercially available products of the mixture of the silicone resin (A1-1) and the silicone resin (A1-2) include DOWSIL MQ-1640 Flake Resin (a mixture of trimethylsiloxysilicate and polypropylsilsesquioxane, manufactured by Dow Toray Co., Ltd.).

(Component (A2))

The component (A2) is a silicone polymer containing a polysiloxane moiety and a moiety formed of a non-silicone organic chain. The non- silicone organic monomer to constitute the moiety formed of a non-silicone organic chain is, from the viewpoint of availability on the market, preferably selectable from a radical-polymerizable ethylenicallyunsaturated monomer, a polycondensation-polymerizable monomer (e.g., those to form polyamides, polyesters or polyurethanes), and a ring-cleavable monomer (e.g., oxazoline or caprolactone-type ones).

Preferably, the component (A2) includes those containing one or more selected from the group consisting of the following components (A2-1) to (A2-4), more preferably those containing the component (A2-1).

(A2-1) An acryl silicone polymer.

(A2-2) A silicone-modified alicyclic structure-containing polymer.

(A2-3) A silicone-modified pullulan.

(A2-4) A polyurea/urethane silicone.

[Acryl Silicone Polymer (A2-1)]

The acryl silicone polymer of the component (A2-1) includes an acrylic polymer having a carbosiloxane dendrimer structure in the side chain, an acryl-silicone graft copolymer, and a graft-type copolymer or alternate block-type copolymer where a structural unit of a polysiloxane group and a structural unit of a polymer of an unsaturated monomer via a sulfide bond.

The acrylic polymer having a carbosiloxane dendrimer structure in the side chain includes a silicone dendrimer-acryl copolymer, and for example, can be produced according to the production method described in JP H11-1530 A and JP 2000-63225 A.

The acrylic polymer having a carbosiloxane dendrimer structure in the side chain is preferably, as INCI nomenclature, acrylates/polytrimethylsiloxymethacrylate copolymer. Commercially available products thereof include DOWSII, FA 4001 CM Silicone Acrylate (30% by mass decamethylcyclopentasiloxane solution), DOWSII, FA 4002 ID Silicone Acrylate (40% by mass isododecane solution), DOWSIL FA 4003 DM Silicone Acrylate (40% by mass dimethicone solution), DOWSIL FA 4004 ID Silicone Acrylate (40% by mass isododecane solution), and DOWSIL FA 4103 Silicone Acrylate Emulsion (30% by mass aqueous solution) (all manufactured by Dow Toray Co., Ltd.).

The acryl-silicone graft copolymer includes a radical polymer of an organopolysiloxane compound having a radical polymerizable group at one terminal of the molecular chain and a radical polymerizable monomer mainly composed of an acrylate and/or a methacrylate.

Examples of the radical polymer of an organopolysiloxane compound having a radical polymerizable group at one terminal of the molecular chain and a radical polymerizable monomer mainly composed of an acrylate and/or a methacrylate usable here include those described in JP H2-25411 A and JP H2-132141 A, and acryl-silicone graft copolymers described in JP H3-162442 A and JP 2003-104825 A.

The acryl-silicone graft copolymer is preferably, as INCI nomenclature, (acrylates/dimethicone) copolymer. Commercially available products thereof include KP-545 (30% by mass decamethylcyclopentasiloxane solution), KP-549 (40% by mass methyltrimethicone solution), and KP-550 (40% by mass isododecane solution) (all manufactured by Shin-Etsu Chemical Co., Ltd.).

The graft-type copolymer or alternate block-type copolymer where a structural unit of a polysiloxane group and a structural unit of a polymer of an unsaturated monomer via a sulfide bond include graft-type copolymers or alternate block-type copolymers described in JP H6-92825 A.

Above all, from the viewpoint of film formability and improving the durability of the treatment effect of the keratin substance or the fiber for a head decoration product, the component (A2-1) is preferably one or more selected from the group consisting of an acrylic polymer having a carbosiloxane dendrimer structure in the side chain and an acryl-silicone graft copolymer, more preferably one or more selected from (acrylates/polytrimethylsiloxymethacrylate) copolymer and (acrylates/dimethicone) copolymer, and even more preferably (acrylates/dimethicone) copolymer.

[Silicone-Modified Alicyclic Structure-Containing Polymer (A2-2)]

Examples of the silicone-modified alicyclic structure-containing polymer include silicone-modified cyclic polyolefins, and preferred examples thereof include silicone-modified polynorbornenes represented by the following general formula (A2-2-1).

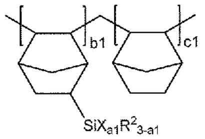

(A2-2-1)

In the formula, each $R^2$ independently represents an alkyl group having 1 or more and 12 or less carbon atoms, X represents a group represented by the following formula (i), a1 is an integer of 1 or more and 3 or less, b1 and c1 each are a repeating unit number, and are each independently an integer of 1 or more.

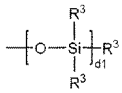

(i)

In the formula, each $R^3$ independently represents a hydrocarbon group having 1 or more and 12 or less carbon atoms, and d1 is an integer of 1 or more and 5 or less.

In the general formula (A2-2-1), $R^2$ is preferably a methyl group, an ethyl group, a n-propyl group, a butyl group, or a pentyl group, and more preferably a methyl group.

X is a group represented by the formula (i), and in the formula (i), each $R^3$ independently is a hydrocarbon group having 1 or more and 12 or less carbon atoms. $R^3$ is preferably an alkyl group having 1 or more and 12 or less carbon atoms or a phenyl group, more preferably an alkyl group having 1 or more and 3 or less carbon atoms, and even more preferably a methyl group. d1 is an integer of 1 or more and 5 or less, and is, from the viewpoint of versatility, preferably d1 = 1. Specifically, X is preferably a trimethylsiloxy group.

a1 is an integer of 1 or more and 3 or less, and, for example, in the polymer, a repeating unit of a1 = 2 and a repeating unit of a1 = 3 may exist as mixed. From the viewpoint of versatility, a1 is preferably 3.

The proportion of b1 and c1 in the general formula (A2-2-1) is preferably b1/c1 = 20/80 to 90/10 (mol/mol), more preferably 30/70 to 80/20 (mol/mol), and even more preferably 50/50 to 70/30 (mol/mol). The proportion of b1 and c1 can be determined by $^1$H-NMR measurement.

The silicone-modified polynorbornene is preferably a silicone-modified polynorbornene represented by the following formula (A2-2-2).

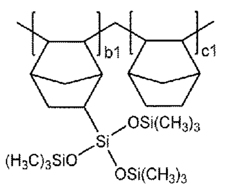

(A2-2-2)

In the formula, b1 and c1 are the same as above.

The silicone-modified polynorbornene represented by the formula (A2-2-2) include a compound of, as INCI nomenclature, (norbornene/tris(trimethylsiloxy)silylnorbornene copolymer).

Commercially available products of the silicone-modified polynorbornene include NBN-30-ID (isododecane solution of (norbornene/tris(trimethylsiloxy)silylnorbomene) copolymer) (by Shin-Etsu Chemical Co., Ltd.).

[Silicone-Modified Pullulan (A2-3)]

The silicone-modified pullulan includes a pullulan having a silicone structure in the side chain, and specifically preferred is a silicone-modified pullulan in which at least a part of the hydrogen atoms of the OH groups in pullulan are substituted with a group represented by the following general formula (ii).

$$-R^4\text{-}SiX_{a1}R^2_{3\text{-}a1} \qquad \text{(ii)}$$

In the formula, $R^4$ represents a single bond or a divalent organic group, and $R^2$, X and a1 are the same as above. From the viewpoint of versatility, X is preferably a trimethylsiloxyl group, and a1 is preferably 3.

In the general formula (ii), $R^4$ is preferably a divalent organic group, more preferably a divalent group represented by the following general formula (iii) or (iv), and even more preferably a divalent group represented by the general formula (iv).

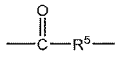

(iii)

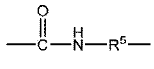

(iv)

In the formulae, $R^5$ represents an alkylene group having 1 or more and 10 or less carbon atoms, and examples thereof include a methylene group, an ethylene group, a trimethylene group, a propylene group and a butylene group. Among these, preferred are an ethylene group, a trimethylene group and a propylene group; and more preferred are a

trimethylene group and a propylene group.

Commercially available products of the silicone-modified pullulan include TSPL-30-ID (isododecane solution of tri(trimethylsiloxy)silylpropylcarbamate pullulan), and TSPL-30-D5 (cyclopentasiloxane solution of tri(trimethylsiloxy)silylpropylcarbamate pullulan) (all manufactured by Shin-Etsu Chemical Co., Ltd.).

[Polyurea/Urethane Silicone (A2-4)]

The polyurea/urethane silicone of the component (A2-4) includes a polysiloxane/polyurea/polyurethane block terpolymer. For example, it is a dimethylpolysiloxane/urea copolymer of "polyurea-dimethicone" as INCI nomenclature.

The polymer can be produced by copolymerization of an $\alpha,\omega$-aminosilicone and a diisocyanate. Commercially available products of the polyurea/urethane silicone include Wacker-Belsil UD 60, Wacker-Belsil UD 80, Wacker-Belsil UD 140, and Wacker-Belsil UD 200 (all manufactured by Wacker Corporation).

One or more can be used as the component (A). Among the above, from the viewpoint of film formability and improving the durability of the treatment effect of the keratin substance or the fiber for a head decoration product, the component (A) preferably contains one or more selected from the group consisting of the component (A1), the component (A2-1), and the component (A2-2), more preferably one or more selected from the group consisting of the component (A1) and the component (A2-1), still more preferably contains one or more selected from the group consisting of the component (A1-2) and the component (A2-1), and even more preferably the component (A1-2).

In addition, both the component (A1) and the component (A2) may be contained as the component (A). In this case, the component (A) preferably contains the component (A1) and one or more selected from the group consisting of the component (A2-1) and the component (A2-2), and more preferably contains the component (A1-2) and the component (A2-1).

In a case where the component (A) contains both the component (A1) and the component (A2), the content of the component (A1) with respect to the total content of the component (A1) and the component (A2) is preferably 0.50 or more and more preferably 0.60 or more in terms of a mass ratio [(A1)/{(A1) + (A2)}] from the viewpoint of film formability, and is preferably 0.90 or less, more preferably 0.85 or less, and even more preferably 0.80 or less from the viewpoint of improving the durability of the treatment effect of the keratin substance or the fiber for a head decoration product. Accordingly, the content of the component (A1) with respect to the total content of the component (A1) and the component (A2) is preferably 0.50 or more and 0.90 or less, more preferably 0.50 or more and 0.85 or less, and even more preferably 0.60 or more and 0.80 or less in terms of a mass ratio [(A1)/{(A1) + (A2)}].

More specifically, from the viewpoint of film formability and improving the durability of the treatment effect of the keratin substance or the fiber for a head decoration product, the component (A) preferably contains one or more selected from the group consisting of trimethylsiloxysilicate, phenylpropyldimethylsiloxysilicate, trifluoropropyldimethyltrimethylsiloxysilicate, (trimethylsiloxysilicate/dimethiconol) crosspolymer, polymethylsilsesquioxane, polypropylsilsesquioxane, (acrylates/polytrimethylsiloxymethacrylate) copolymer, (acrylates/dimethicone) copolymer, and (norbornene/tris(trimethylsiloxy)silylnorbornene) copolymer, more preferably contains one or more selected from the group consisting of trimethylsiloxysilicate, phenylpropyldimethylsiloxysilicate, trifluoropropyldimethyltrimethylsiloxysilicate, (trimethylsiloxysilicate/dimethiconol) crosspolymer, polymethylsilsesquioxane, polypropylsilsesquioxane, (acrylates/polytrimethylsiloxymethacrylate) copolymer, and (acrylates/dimethicone) copolymer, still more preferably contains one or more selected from the group consisting of trimethylsiloxysilicate and (acrylates/dimethicone) copolymer, even more preferably contains trimethylsiloxysilicate, and even more preferably is trimethylsiloxysilicate.

(Component (B): Organopolysiloxane other than Component (A), which has a cationic group)

The first agent further contains, as a component (B), an organopolysiloxane other than the component (A), which has a cationic group, from the viewpoint of enhancing the durability of the treatment effect of the keratin substance or the fiber for a head decoration product and improving the feel. It is considered that the component (B) can improve the feel of the keratin substance or the fiber for a head decoration product after treatment with the first agent because it has a cationic group. In addition, since the component (B) has high adsorptivity to the keratin substance or the fiber for a head decoration product, it is considered that the component (B) is unevenly distributed on the keratin substance or the fiber for a head decoration product side in the film formed, strongly adheres the film to the surface thereof, and enhances the peeling prevention effect of the film.

The cationic group contained in the component (B) refers to a cationic group or a group which can be ionized to become a cationic group. Specific examples thereof include a primary amino group, a secondary amino group, a tertiary amino group, and a quaternary ammonium group, and from the viewpoint of adsorptivity to the surface of the keratin substance or the fiber for a head decoration product, the cationic group is preferably one or more selected from the group consisting of a primary amino group, a secondary amino group, and a tertiary amino group.

As the component (B), one or more selected from the group consisting of an amino-modified silicone (B 1) and

an aminopolyether-modified silicone (B2) are preferable from the viewpoint of enhancing the durability of the treatment effect of the keratin substance or the fiber for a head decoration product and improving the feel.

[Component (B 1): Amino-modified Silicone]

In the present invention, the amino-modified silicone (B 1) is a silicone other than the component (A), which has an amino group and does not have a polyether moiety, and is preferably a silicone represented by the following general formula (1).

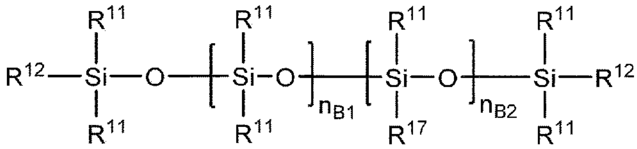
(1)

In the formula, each $R^{11}$ independently represents a hydrocarbon group having 1 or more and 6 or less carbon atoms, each $R^{12}$ independently represents a hydroxy group, an alkoxy group having 1 or more and 6 or less carbon atoms, or a hydrocarbon group having 1 or more and 22 or less carbon atoms, $R^{17}$ represents a primary to tertiary amino group-containing group, $n_{B1}$ represents a number of 0 or more, $n_{B2}$ represents a number of 1 or more, $n_{B1}+n_{B2}$ represents a degree of polymerization, and $n_{B2}$'s $R^{17}$s may be the same as or different from each other.

In the general formula (1), the hydrocarbon group in $R^{11}$ may be either an aliphatic group or an aromatic group, and examples thereof include an alkyl group, an alkenyl group, and a phenyl group. The alkyl group and the alkenyl group may be linear or branched.

Among the above, $R^{11}$ is preferably an alkyl group having 1 or more and 6 or less carbon atoms or a phenyl group, more preferably an alkyl group having 1 or more and 3 or less carbon atoms or a phenyl group, still more preferably a methyl group or a phenyl group, and even more preferably a methyl group.

In the general formula (1), each $R^{12}$ independently represents a hydroxy group, an alkoxy group having 1 or more and 6 or less carbon atoms, or a hydrocarbon group having 1 or more and 22 or less carbon atoms.

Examples of the alkoxy group in $R^{12}$ include a methoxy group, an ethoxy group, a propoxy group, and a butoxy group.

The hydrocarbon group in $R^{12}$ is preferably an alkyl group having 1 or more and 22 or less carbon atoms or a phenyl group, and more preferably an alkyl group having 1 or more and 18 or less carbon atoms or a phenyl group.

From the viewpoint of enhancing the durability of the treatment effect of the keratin substance or the fiber for a head decoration product and providing a good feel, $R^{12}$ is preferably a hydroxy group, an alkyl group having 1 or more and 22 or less carbon atoms, or a phenyl group, more preferably an alkyl group having 1 or more and 20 or less carbon atoms, still more preferably a methyl group, an ethyl group, a propyl group, a butyl group, a hexyl group, an octyl group, a decyl group, a dodecyl group, a cetyl group, a hexadecyl group, a stearyl group, or an eicosanyl group, and even more preferably a methyl group, a cetyl group, a stearyl group, or an eicosanyl group.

In the general formula (1), $R^{17}$ represents a primary to tertiary amino group-containing group.

The primary to tertiary amino group-containing group in $R^{17}$ is preferably a group represented by $-N(R^{18})_2$, $-NR^{18}(CH_2)_qN(R^{18})_2$, or $-NR^{18}(CH_2)_qN(R^{19})CO-R^{20}$. Here, $R^{18}$ represents a hydrogen atom or a monovalent hydrocarbon group having 1 or more and 4 or less carbon atoms, and preferably a hydrogen atom, a methyl group or an ethyl group. $R^{19}$ represents a monovalent hydrocarbon group having 1 or more and 4 or less carbon atoms, and is preferably a methyl group or an ethyl group. $R^{20}$ represents a monovalent hydrocarbon group having 1 or more and 4 or less carbon atoms. q is a number of 2 or more and 6 or less, and is preferably a number of 2 or more and 4 or less.

The amino group-containing group in $R^{17}$ is preferably $-(CH_2)_3-NH_2$, $-(CH_2)_3-N(CH_3)_2$, $-(CH_2)_3-NH-(CH_2)_2-NH_2$, or $-(CH_2)_2-NH-(CH_2)_2-N(CH_3)_2$, and more preferably $-(CH_2)_3-NH_2$, or $-(CH_2)_3-NH-(CH_2)_2-NH_2$.

In the general formula (1), $n_{B1}$ is a number of 0 or more, and $n_{B2}$ is a number of 1 or more. $n_{B1}+n_{B2}$ represents a degree of polymerization. From the viewpoint of enhancing the durability of the treatment effect of the keratin substance or the fiber for a head decoration product and providing a good feel, $n_{B1}+n_{B2}$ is preferably a number of 5 or more.

Examples of commercially available products of the amino-modified silicone used as the component (B1) include KF-8004, KF-8005S (aminoethylaminopropyl dimethicone), KF-8015 (aminopropyl dimethicone) (all manufactured by Shin-Etsu Chemical Co., Ltd.), XF42-B1989 (amodimethicone (aminoethylaminopropylmethylsiloxane-dimethylsiloxane copolymer)), and Silsoft AX (bis-cetearyl aminomethicone) (all manufactured by Momentive Performance Materials Japan LLC).

[Component (B2): Aminopolyether-modified silicone]

In the present invention, the aminopolyether-modified silicone (B2) is a silicone other than the component (A), which has an amino group and a polyether moiety. In the component (B2), the amino group and the polyether moiety may be present in the silicone main chain or may be present in the side chain portion.

More specifically, the component (B2) is more preferably one or more selected from the group consisting of an aminopolyether-modified silicone (B2-1) having a repeating unit represented by the following general formula (2) and an aminopolyether-modified silicone (B2-2) having a repeating unit represented by the following general formula (3).

In the following description, the aminopolyether-modified silicone (B2-1) having a repeating unit represented by the general formula (2) is also referred to as "component (B2-1)", and the aminopolyether-modified silicone (B2-2) having a repeating unit represented by the general formula (3) is also referred to as "component (B2-2)".

<<Component (B2-1)>>

The component (B2-1) is an aminopolyether-modified silicone having a repeating unit represented by the following general formula (2).

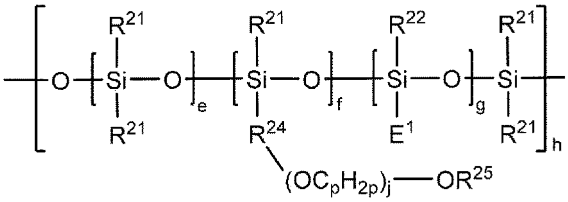

(2)

In the formula (2), $R^{21}$ independently represents a monovalent hydrocarbon group having 1 or more and 6 or less carbon atoms, $R^{22}$ represents any of $R^{21}$ or $E^1$, $E^1$ represents a monovalent group represented by $-R^{23}-Z^1$ (where $R^{23}$ represents a divalent hydrocarbon group having 1 or more and 6 or less carbon atoms, and $Z^1$ represents a primary to tertiary amino group-containing group), $R^{24}$ represents a divalent hydrocarbon group having 1 or more and 6 or less carbon atoms, $R^{25}$ represents a hydrogen atom or a monovalent hydrocarbon group having 1 or more and 4 or less carbon atoms.

e is a number of 1 or more and 50 or less, f is a number of 1 or more and 50 or less, g is a number of 1 or more and 50 or less, h is a number of 1 or more, j is a number of 2 or more and 100 or less, p is a number of 2 or more and 10 or less. The bonding order of the parenthesized structural units is not limited, and the bonding form thereof may be in a block form or a random form. j's $(OC_pH_{2p})$s can be the same or different. Plural $R^{21}$'s, $R^{22}$'s, $R^{24}$'s, $R^{25}$'s and $E^1$'s can be the same or different.

In the general formula (2), $R^{21}$ represents a monovalent hydrocarbon group having 1 or more and 6 or less carbon atoms, and is preferably an alkyl group having 1 or more and 6 or less carbon atoms, or a phenyl group, more preferably a methyl group or an ethyl group, and even more preferably a methyl group. $R^{22}$ is any of $R^{21}$ or $E^1$, and is preferably $R^{21}$.

In the general formula (2), $E^1$ represents a monovalent group represented by $-R^{23}-Z^1$ (where $R^{23}$ represents a divalent hydrocarbon group having 1 or more and 6 or less carbon atoms, and $Z^1$ represents a primary to tertiary amino group-containing group). $R^{23}$ is preferably a divalent hydrocarbon group having 2 or more and 4 or less carbon atoms, and more preferably an ethylene group, a trimethylene group, a propylene group or a tetramethylene group.

$Z^1$ is a primary to tertiary amino group-containing group, and is preferably a group represented by $-N(R^{26})_2$, $-NR^{26}(CH_2)_qN(R^{26})_2$, or $-NR^{26}(CH_2)_qN(R^{27})CO-R^{28}$. Here, $R^{26}$ represents a hydrogen atom or a monovalent hydrocarbon group having 1 or more and 4 or less carbon atoms, and preferably a hydrogen atom, a methyl group or an ethyl group. $R^{27}$ represents a monovalent hydrocarbon group having 1 or more and 4 or less carbon atoms, and is preferably a methyl group or an ethyl group. $R^{28}$ represents a monovalent hydrocarbon group having 1 or more and 4 or less carbon atoms. q is a number of 2 or more and 6 or less, and is preferably a number of 2 or more and 4 or less.

In the general formula (2), the group $E^1$ is preferably $-(CH_2)_3-NH_2$, $-(CH_2)_3-N(CH_3)_2$, $-(CH_2)_3-NH-(CH_2)_2-NH_2$, or $-(CH_2)_2-NH-(CH_2)_2-N(CH_3)_2$, and more preferably $-(CH_2)_3-NH_2$, or $-(CH_2)_3-NH-(CH_2)_2-NH_2$.

In the general formula (2), $R^{24}$ is a divalent hydrocarbon group having 1 or more and 6 or less carbon atoms, preferably a divalent hydrocarbon group having 2 or more and 4 or less carbon atoms, and more preferably an ethylene group, a trimethylene group, a propylene group or a tetramethylene group.

$R^{25}$ is a hydrogen atom or a monovalent hydrocarbon group having 1 or more and 4 or less carbon atoms, and is preferably a methyl group or an ethyl group.

In the general formula (2), e is a number of 1 or more and 50 or less, f is a number of 1 or more and 50 or less,

g is a number of 1 or more and 50 or less, h is a number of 1 or more, and j is a number of 2 or more and 100 or less. p is a number of 2 or more and 10 or less, and is preferably 2 or more and 6 or less, and more preferably 2 or more and 4 or less.

In particular, the component (B2-1) is more preferably one represented by the following general formula (2-1).

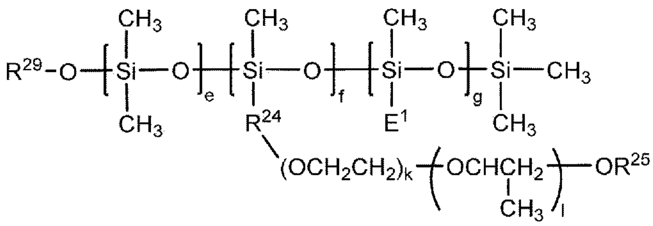
(2-1)

In the formula (2-1), $E^1$, $R^{24}$, $R^{25}$, e, f, and g are the same as above, $R^{29}$ represents a monovalent hydrocarbon group having 1 or more and 4 or less carbon atoms, or a trimethylsilyl group, k is a number of 1 or more and 50 or less, l is a number of 0 or more and 50 or less, and is preferably a number of 1 or more and 50 or less.

$R^{29}$ is preferably a methyl group, an ethyl group or a trimethylsilyl group, and more preferably a trimethylsilyl group.

As the component (B2-1), one or more can be used either singly or as combined.

As the component (B2-1), commercially-available aminopolyether-modified silicones can be used. Examples thereof include ABIL Soft AF10 (aminopolyether-modified silicone represented by the general formula (2-1) (methoxy PEG/PPG-7/3 aminopropyl dimethicone)) (manufactured by Evonik Corporation).

(Component (B2-2))

The component (B2-2) is an aminopolyether-modified silicone having a repeating unit represented by the following general formula (3).

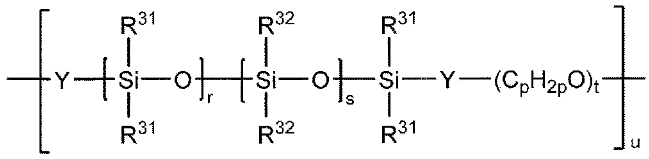
(3)

In the formula (3), $R^{31}$ represents a monovalent hydrocarbon group having 1 or more and 6 or less carbon atoms, $R^{32}$ represents any of $R^{31}$ or $E^2$, $E^2$ represents a monovalent group represented by $-R^{33}-Z^2$ (where $R^{33}$ represents a single bond, or a divalent hydrocarbon group having 1 or more and 20 or less carbon atoms, and $Z^2$ represents a primary to tertiary amino group-containing group), Y represents a single bond, or a divalent group having 1 or more and 12 or less carbon atoms. In the case where all $R^{32}$'s are $R^{31}$'s, at least one Y is an amino group-containing divalent group. In the case where all Y's are divalent groups not containing an amino group, at least one $R^{32}$ is $E^2$.

r is a number of 1 or more, s is a number of 1 or more, t is a number of 2 or more and 100 or less, and u is a number of 1 or more. p is the same as above, and is a number of 2 or more and 10 or less. The bonding order of the parenthesized structural units is not limited, and the bonding form thereof may be in a block form or a random form. t's $(C_pH_{2p}O)$s can be the same or different. Plural $R^{31}$'s, $R^{32}$'s, $E^2$'s and Y's can be the same or different.

In the general formula (3), $R^{31}$ represents a monovalent hydrocarbon group having 1 or more and 6 or less carbon atoms, and is independently preferably an alkyl group having 1 or more and 6 or less carbon atoms, or a phenyl group, more preferably a methyl group or an ethyl group, even more preferably a methyl group.

In the general formula (3), $R^{32}$ is any of $R^{31}$ or $E^2$. $E^2$ represents a monovalent group represented by $-R^{33}-Z^2$ (where $R^{33}$ represents a single bond, or a divalent hydrocarbon group having 1 or more and 20 or less carbon atoms).

$R^{33}$ is preferably a divalent hydrocarbon group having 1 or more and 20 or less carbon atoms, more preferably an alkylene group having 1 or more and 20 or less carbon atoms, still more preferably a linear or branched alkylene group having 1 or more and 6 or less carbon atoms, even more preferably a methylene group, an ethylene group, a trimethylene group, a propylene group, a tetramethylene group, or a hexamethylene group, and even more preferably a trimethylene group or a propylene group.

$Z^2$ is a primary to tertiary amino group-containing group, and is preferably an amino group-containing group represented by $-N(R^{34})_2$, $-NR^{34}(CH_2)_qN(R^{34})_2$, or $-NR^{34}(CH_2)_qN(R^{35})CO-R^{36}$. Here, $R^{34}$ and $R^{35}$ each independently

represent a hydrogen atom or an alkyl group having 1 or more and 3 or less carbon atoms, and is preferably a hydrogen atom or a methyl group. $R^{36}$ represents an alkyl group having 1 or more and 3 or less carbon atoms. q is a number of 1 or more and 6 or less, and is preferably a number of 2 or more and 4 or less.

In the general formula (3), the group $E^2$ is preferably $-(CH_2)_3-NH_2$, $-(CH_2)_3-N(CH_3)_2$, $-(CH_2)_3-NH-(CH_2)_2-NH_2$, or $-(CH_2)_2-NH-(CH_2)_2-N(CH_3)_2$, and more preferably $-(CH_2)_3-NH_2$, or $-(CH_2)_3-NH-(CH_2)_2-NH_2$.

In the general formula (3), Y is a single bond, or a divalent group having 1 or more and 12 or less carbon atoms. The divalent group having 1 or more and 12 or less carbon atoms is preferably an alkylene group having 1 or more and 6 or less carbon atoms, an alkyleneoxy group having 1 or more and 6 or less carbon atoms, or a divalent group represented by $-R^{37}-O-CH_2-CH(OH)-CH_2-N(R^{38})-R^{39}-O-$ of an amino group-containing divalent group. Here, $R^{37}$ and $R^{39}$ each independently represent an alkylene group having 1 or more and 6 or less carbon atoms, preferably an alkylene group having 2 or more and 4 or less carbon atoms, and more preferably a propylene group. $R^{38}$ represents a hydrogen atom or an alkyl group having 1 or more and 3 or less carbon atoms.

In the general formula (3), the alkylene group having 1 or more and 6 or less carbon atoms and the alkylene group in the alkyleneoxy group having 1 or more and 6 or less carbon atoms for Y are preferably an ethylene group, a propylene group, a trimethylene group, a n-butylene group (tetramethylene group) or an isobutylene group, and more preferably a n-butylene group or an isobutylene group. The isobutylene group as referred to herein includes $-CH(CH_3)CH_2CH_2-$, $-CH_2CH(CH_3)CH_2-$, and $-CH_2CH_2CH(CH_3)-$.

In the general formula (3), r is a number of 1 or more, s is a number of 1 or more, t is a number of 2 or more and 100 or less, and u is a number of 1 or more. r is preferably a number of 1 or more and 1000 or less, and more preferably a number of 2 or more and 200 or less. s is preferably a number of 1 or more and 100 or less, t is preferably a number of 4 or more and 80 or less, and more preferably 10 or more and 50 or less, u is preferably a number of 1 or more and 300 or less, and more preferably a number of 1 or more and 150 or less.

In the general formula (3), p is a number of 2 or more and 10 or less, preferably 2 or more and 6 or less, and more preferably 2 or more and 4 or less.

The component (B2-2) is more preferably one or more selected from the group consisting of an aminopolyether-modified silicone having a structure represented by the following general formula (3-1) and an aminopolyether-modified silicone having a structure represented by the following general formula (3-2). Still more preferably, the component (B2-2) is one or more selected from the group consisting of an aminopolyether-modified silicone composed of a structure represented by the following general formula (3-1) and an aminopolyether-modified silicone composed of a structure represented by the following general formula (3-2), and even more preferably an aminopolyether-modified silicone composed of a structure represented by the following general formula (3-1).

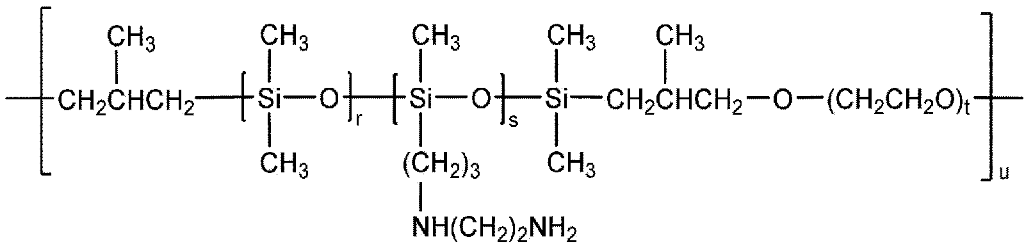

(3-1)

In the formula (3-1), r, s, t and u are the same as above.

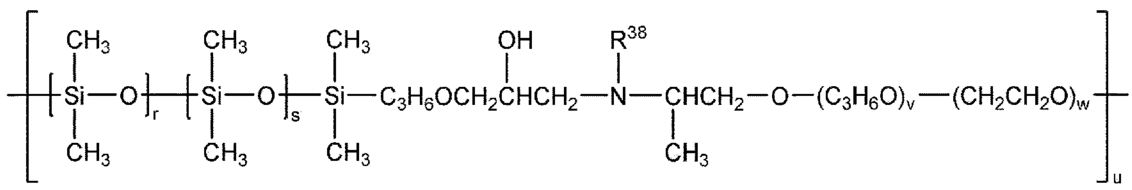

(3-2)

In the formula (3-2), $R^{38}$, r, s and u are the same as above, v is a number of 0 or more and 50 or less, and preferably a number of 2 or more and 50 or less. w is a number of 2 or more and 100 or less. v + w is a number of 2 or more and 100 or less, preferably a number of 4 or more and 80 or less, and more preferably a number of 10 or more and 50 or less.

As the component (B2-2), one or more can be used either singly or as combined.

As the component (B2-2), commercially-available aminopolyether-modified silicones can be used. Examples thereof include, as the aminopolyether-modified silicone having a structure represented by the general formula (3-1), DOWSIL SS-3588 Fluid (80% by mass ethanol solution of (bisisobutyl-PEG-15/amodimethicone) copolymer), DOWSIL

SILSTYLE 104 ((bisisobutyl-PEG-14/amodimethicone) copolymer), DOWSIL SILSTYLE 201 ((bisisobutyl-PEG-14/amodimethicone) copolymer), and DOWSIL SILSTYLE 401((bisisobutyl PEG/PPG-20/35/amodimethicone) copolymer) (all manufactured by Dow Toray Co., Ltd.). In addition, examples of the aminopolyether-modified silicone having a structure represented by the general formula (3-2) include Silsoft A+ (PEG-40/PPG-8 methylaminopropyl/hydroxypropyldimethicone copolymer) (manufactured by Momentive Performance Materials, Inc.).

As the component (B), one or more of the above can be used. In particular, from the viewpoint of enhancing the durability of the treatment effect of the keratin substance or the fiber for a head decoration product and improving the feel, the component (B) is preferably one or more selected from the group consisting of an amino-modified silicone represented by the above general formula (1) and an aminopolyether-modified silicone (B2-2) having a repeating unit represented by the above general formula (3), more preferably one or more selected from the group consisting of an amino-modified silicone represented by the above general formula (1) and an aminopolyether-modified silicone composed of a structure represented by the above general formula (3-1), and even more preferably an aminopolyether-modified silicone composed of a structure represented by the above general formula (3-1).

Further, from the viewpoint of removability of the film by step (II) and from the viewpoint of improving the quick-drying property and the feel of the keratin substance or the fiber for a head decoration product after step (II), the component (B) preferably includes one or more selected from the group consisting of bis-cetearyl amodimethicone and an aminopolyether-modified silicone (B2), more preferably includes one or more selected from the group consisting of bis-cetearyl amodimethicone and an aminopolyether-modified silicone (B2-2) having a repeating unit represented by the general formula (3), still more preferably includes one or more selected from the group consisting of bis-cetearyl amodimethicone and an aminopolyether-modified silicone of a structure represented by the general formula (3-1), and even more preferably is one or more selected from the group consisting of bis-cetearyl amodimethicone and an aminopolyether-modified silicone of a structure represented by the general formula (3-1).

(Content)

The content of the component (A) in the first agent is preferably 0.05% by mass or more, more preferably 0.1% by mass or more, still more preferably 0.5% by mass or more, even more preferably 1% by mass or more, even more preferably 2% by mass or more, even more preferably 3% by mass or more, and even more preferably 5% by mass or more, from the viewpoint of film formability in the step (I) and improving the durability of the treatment effect of the keratin substance or the fiber for a head decoration product, and from the viewpoint of improving the quick-drying property and the feel of the keratin substance or the fiber for a head decoration product after the step (II). On the other hand, from the viewpoint of improving the feel of the keratin substance or the fiber for a head decoration product by the step (I) and from the viewpoint of the removability of the film by the step (II), the content thereof is preferably 30% by mass or less, more preferably 25% by mass or less, and still more preferably 23% by mass or less. Thus, the content of the component (A) in the first agent is preferably 0.05% by mass or more and 30% by mass or less, more preferably 0.1% by mass or more and 25% by mass or less, still more preferably 0.5% by mass or more and 25% by mass or less, even more preferably 1% by mass or more and 25% by mass or less, even more preferably 2% by mass or more and 25% by mass or less, even more preferably 3% by mass or more and 23% by mass or less, and even more preferably 5% by mass or more and 23% by mass or less.

The content of the component (B) in the first agent is preferably 0.01% by mass or more, more preferably 0.02% by mass or more, still more preferably 0.03% by mass or more, and even more preferably 0.05% by mass or more, from the viewpoint of enhancing the durability of the treatment effect of the keratin substance or the fiber for a head decoration product by the step (I) and improving the feel, and is preferably 30% by mass or less, more preferably 25% by mass or less, still more preferably 20% by mass or less, even more preferably 15% by mass or less, even more preferably 10% by mass or less, even more preferably 5% by mass or less, even more preferably 3% by mass or less, and even more preferably 2% by mass or less, from the viewpoint of the removability of the film by the step (II) and from the viewpoint of improving the quick-drying property and the feel of the keratin substance or the fiber for a head decoration product after the step (II). Thus, the content of the component (B) in the first agent is preferably 0.01% by mass or more and 30% by mass or less, more preferably 0.02% by mass or more and 25% by mass or less, still more preferably 0.03% by mass or more and 20% by mass or less, even more preferably 0.03% by mass or more and 15% by mass or less, even more preferably 0.05% by mass or more and 10% by mass or less, even more preferably 0.05% by mass or more and 5% by mass or less, even more preferably 0.05% by mass or more and 3% by mass or less, and even more preferably 0.05% by mass or more and 2% by mass or less.

The total content of the component (A) and the component (B) in the first agent is preferably 0.06% by mass or more, more preferably 0.1% by mass or more, still more preferably 0.5% by mass or more, even more preferably 1% by mass or more, even more preferably 2% by mass or more, and even more preferably 5% by mass or more, from the viewpoint of film formability in the step (I) and improving the durability of the treatment effect of the keratin substance or the fiber for a head decoration product, from the viewpoint of the removability of the film by the step (II), and from the

viewpoint of improving the quick-drying property and the feel of the keratin substance or the fiber for a head decoration product after the step (II). On the other hand, from the viewpoint of ease of application to the keratin substance or the fiber for a head decoration product, the total content thereof is preferably 50% by mass or less, more preferably 45% by mass or less, still more preferably 40% by mass or less, even more preferably 30% by mass or less, and even more preferably 25% by mass or less. Thus, the total content of the component (A) and the component (B) in the first agent is preferably 0.06% by mass or more and 50% by mass or less, more preferably 0.1% by mass or more and 45% by mass or less, still more preferably 0.5% by mass or more and 40% by mass or less, even more preferably 1% by mass or more and 30% by mass or less, even more preferably 2% by mass or more and 25% by mass or less, and even more preferably 5% by mass or more and 25% by mass or less.

The mass ratio of the content of the component (B) to the content of the component (A) [(B)/(A)] in the first agent is preferably 0.005 or more, and more preferably 0.010 or more, from the viewpoint of film formability in the step (I) and improving the durability of the treatment effect of the keratin substance or the fiber for a head decoration product, from the viewpoint of improving the removability of the film by the step (II), and from the viewpoint of improving the quick-drying property and the feel of the keratin substance or the fiber for a head decoration product after the step (II). On the other hand, from the viewpoint of the removability of the film by the step (II), and from the viewpoint of improving the quick-drying property and the feel of the keratin substance or the fiber for a head decoration product after the step (II), it is preferably 50 or less, more preferably 30 or less, still more preferably 20 or less, even more preferably 10 or less, even more preferably 5 or less, even more preferably 3 or less, even more preferably 1 or less, and even more preferably 0.8 or less. Thus, the mass ratio of the content of the component (B) to the content of the component (A) [(B)/(A)] in the first agent is preferably 0.005 or more and 50 or less, more preferably 0.010 or more and 50 or less, still more preferably 0.010 or more and 30 or less, even more preferably 0.010 or more and 20 or less, even more preferably 0.010 or more and 10 or less, even more preferably 0.010 or more and 5 or less, even more preferably 0.010 or more and 3 or less, even more preferably 0.010 or more and 1 or less, and even more preferably 0.010 or more and 0.8 or less.

(Colorant)

The first agent can further contain a colorant according to the type of the keratin substance or the fiber for a head decoration product to be treated and the intended use. When the first agent is a hair dye, examples of the colorant include a pigment or a dye, and the colorant is preferably a pigment from the viewpoint of ease of application and detachment.

The pigment used in the first agent may be any pigment generally used in makeup cosmetic materials and hair dye compositions, and examples thereof include a white inorganic pigment such as titanium oxide, zinc oxide, cerium oxide and barium sulfate; a colored inorganic pigment such as yellow iron oxide, black iron oxide, red iron oxide, carbon black, chromium oxide, chromium hydroxide, Prussian blue and ultramarine blue; a luster powder such as titanium oxide-coated mica, titanium oxide-coated bismuth oxychloride, iron oxide-coated mica titanium, iron oxide mica, Prussian blue-processed mica titanium, carmineprocessed mica titanium, bismuth oxychloride, and fish scale guanine; an organic pigment such as Red No. 201, Red No. 202, Red No. 205, Red No. 226, Red No. 228, Orange No. 203, Orange No. 204, Blue No. 404, and Yellow No. 401; a lake pigment such as a zirconium, barium or aluminum lake of Red No. 3, Red No. 104, Red No. 106, Orange No. 205, Yellow No. 4, Yellow No. 5, Green No. 3 or Blue No. 1; and a composite pigment such as fine particle titanium oxide-coated mica titanium, fine particle zinc oxide-coated mica titanium, barium sulfate-coated mica titanium, titanium oxide-containing silicon dioxide and zinc oxide-containing silicon dioxide. One or more of these can be used either singly or as combined.

In addition, pigments whose surfaces are treated with various surface treatment agents may be used. The surface treatment is not specifically limited. Various surface treatments can be applied to the pigments, and examples thereof include fluorine compound treatment, silicone treatment, silicone resin treatment, pendant treatment, silane coupling agent treatment, titanium coupling agent treatment, oil treatment, metal soap treatment, N-acylated lysine treatment, polyethylene glycol treatment, PVA treatment, polyacrylic acid treatment, hyaluronic acid treatment, alginic acid treatment, inorganic compound treatment, plasma treatment and mechanochemical treatment.

Examples of the dye used in the first agent include a direct dye and an oxidation dye.

Examples of the direct dye include an acid dye, a nitro dye, a disperse dye, a basic dye, and a direct dye described in JP 2003-342139 A.

Examples of the acidic dye include Blue No. 1, Violet No. 401, Black No. 401, Orange No. 205, Red No. 227, Red No. 106, Yellow No. 203, and Acid Orange 3. Examples of the nitro dye include 2-nitroparaphenylene diamine, 2-amino-6-chloro-4-nitrophenol, 3-nitro-p-hydroxyethylaminophenol, 4-nitroorthophenylene diamine, 4-amino-3-nitrophenol, 4-hydroxypropylamino-3-nitrophenol, HC Blue No. 2, HC Orange No. 1, HC Red No. 1, HC Yellow No. 2, HC Yellow No. 4, HC Yellow No. 5, HC Red No. 3, and N,N-bis-(2-hydroxyethyl)-2-nitroparaphenylene diamine. Examples of the disperse dye include Disperse Violet 1, Disperse Blue 1, and Disperse Black 9. Examples of the basic dye include Basic Blue 99, Basic Brown 16, Basic Brown 17, Basic Red 76, Basic Red 51, Basic Yellow 57, Basic Yellow 87, and Basic

Orange 31. One or more of these direct dyes can be used.

Examples of the oxidation dye include dyes composed of a precursor and a coupler.

Examples of the precursor in the oxidation dye include one or more selected from the group consisting of paraphenylenediamine, toluene-2,5-diamine, 2-chloro-paraphenylenediamine, N-methoxyethyl paraphenylenediamine, N,N-bis(2-hydroxyethyl)paraphenylenediamine, 2-(2-hydroxyethyl)paraphenylenediamine, 2,6-dimethylparaphenylenediamine, 4,4'-diaminodiphenylamine, 1,3-bis(N-(2-hydroxyethyl)-N-(4-aminophenyl)amino)-2-propanol, PEG-3,3,2'-paraphenylenediamine, para-aminophenol, para-methylaminophenol, 3-methyl-4-aminophenol [= 4-aminometacresol], 2-aminomethyl-4-aminophenol, 2-(2-hydroxyethylaminomethyl)-4-aminophenol, ortho-aminophenol, 2-amino-5-methylphenol, 2-amino-6-methylphenol, 2-amino-5-acetamidophenol, 3,4-diaminobenzoic acid, 5-aminosalicylic acid, 2,4,5,6-tetraaminopyrimidine, 2,5,6-triamino-4-hydroxypyrimidine, 4,5-diamino-1-(4'-chlorobenzyl)pyrazole, 1-hydroxyethyl-4,5-diaminopyrazole, and salts thereof.

Examples of the coupler in the oxidation dye include one or more selected from the group consisting of metaphenylenediamine, 2,4-diaminophenoxyethanol, 2-amino-4-(2-hydroxyethylamino)anisole [= 2-amino-4-(β-hydroxyethyl)aminoanisole], 2,4-diamino-5-methylphenetole, 2,4-diamino-5-(2-hydroxyethoxy)toluene, 2,4-dimethoxy-1,3-diaminobenzene, 2,6-bis(2-hydroxyethylamino)toluene, 2,4-diamino-5-fluorotoluene, 1,3-bis(2,4-diaminophenoxy)propane, metaaminophenol, 2-methyl-5-aminophenol [= 5-aminoorthocresol], 2-methyl-5-(2-hydroxyethylamino)phenol, 2,4-dichloro-3-aminophenol, 2-chloro-3-amino-6-methylphenol, 2-methyl-4-chloro-5-aminophenol, N-cyclopentyl-metaaminophenol, 2-methyl-4-methoxy-5-(2-hydroxyethylamino)phenol, 2-methyl-4-fluoro-5-aminophenol, resorcin, 2-methylresorcin, 4-chlororesorcin, 1-naphthol, 1,5-dihydroxynaphthalene, 1,7-dihydroxynaphthalene, 2,7-dihydroxynaphthalene, 2-isopropyl-5-methylphenol, 4-hydroxyindole, 5-hydroxyindole, 6-hydroxyindole, 7-hydroxyindole, 6-hydroxybenzomorpholine, 3,4-methylenedioxyphenol, 2-bromo-4,5-methylenedioxyphenol, 3,4-methylenedioxyaniline, 1-(2-hydroxyethyl)amino-3,4-methylenedioxybenzene, 2,6-dihydroxy-3,4-dimethylpyridine, 2,6-dimethoxy-3,5-diaminopyridine, 2,3-diamino-6-methoxypyridine, 2-methylamino-3-amino-6-methoxypyridine, 2-amino-3 -hydroxypyridine, 2,6-diaminopyridine, and salts thereof.

When the first agent contains a colorant, the content of the colorant in the first agent is preferably 0.01% by mass or more, more preferably 0.05% by mass or more, and still more preferably 0.1% by mass or more, from the viewpoint of imparting desired colorability, and is preferably 50% by mass or less, and more preferably 30% by mass or less, from the viewpoint of dispersibility in the first agent and economic efficiency, and from the viewpoint of maintaining a good feel. Accordingly, the content of the colorant in the first agent is preferably 0.01% by mass or more and 50% by mass or less, more preferably 0.05% by mass or more and 30% by mass or less, and still more preferably 0.1% by mass or more and 30% by mass or less.

(Component (E): Organopolysiloxane other than Component (A) and Component (B))

From the viewpoint of further improving the durability of the treatment effect of the keratin substance or the fiber for a head decoration product, the first agent may further contain an organopolysiloxane other than the component (A) and the component (B) as a component (E).

The degree of polymerization of the component (E) is preferably 100 or more, more preferably 150 or more, still more preferably 250 or more, even more preferably 300 or more, even more preferably 320 or more, and even more preferably 350 or more, from the viewpoint of further improving the durability of the treatment effect of the keratin substance or the fiber for a head decoration product. On the other hand, from the viewpoint of further improving the durability of the treatment effect of the keratin substance or the fiber for a head decoration product, and from the viewpoint of availability, the degree of polymerization is preferably 4,300 or less, more preferably 4,200 or less, still more preferably 4,000 or less, even more preferably 3,800 or less, even more preferably 3,700 or less, and even more preferably 3,650 or less. Accordingly, the degree of polymerization of the component (E) is preferably 100 or more and 4,300 or less, more preferably 150 or more and 4,200 or less, still more preferably 250 or more and 4,200 or less, even more preferably 300 or more and 4,000 or less, even more preferably 320 or more and 3,800 or less, even more preferably 320 or more and 3,700 or less, and even more preferably 350 or more and 3,650 or less.

More specifically, the component (E) is preferably an organopolysiloxane represented by the following general formula (4).

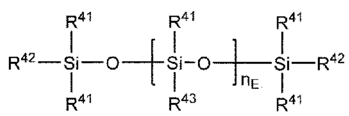 (4)

In the formula, each $R^{41}$ independently represents a hydrocarbon group having 1 or more and 6 or less carbon atoms, each $R^{42}$ independently represents a hydroxy group, an alkoxy group having 1 or more and 6 or less carbon

atoms, or a hydrocarbon group having 1 or more and 6 or less carbon atoms, $R^{43}$ represents a hydrocarbon group having 1 or more and 6 or less carbon atoms, $n_E$ represents a degree of polymerization, and $n_E$ is a number of 100 or more, and $n_E$'s $R^{43}$s may be the same or different from each other.

In the general formula (4), the hydrocarbon group in $R^{41}$ may be either an aliphatic group or an aromatic group, and examples thereof include an alkyl group, an alkenyl group, and a phenyl group. The alkyl group and the alkenyl group may be linear or branched.

Among the above, $R^{41}$ is preferably an alkyl group having 1 or more and 6 or less carbon atoms or a phenyl group, more preferably an alkyl group having 1 or more and 3 or less carbon atoms or a phenyl group, still more preferably a methyl group or a phenyl group, and even more preferably a methyl group.

In the general formula (4), each $R^{42}$ independently represents a hydroxy group, an alkoxy group having 1 or more and 6 or less carbon atoms, or a hydrocarbon group having 1 or more and 6 or less carbon atoms.

Examples of the alkoxy group in $R^{42}$ include a methoxy group, an ethoxy group, a propoxy group, and a butoxy group.

The hydrocarbon group in $R^{42}$ is the same as in $R^{41}$, and is preferably an alkyl group having 1 or more and 6 or less carbon atoms or a phenyl group, more preferably an alkyl group having 1 or more and 3 or less carbon atoms or a phenyl group, still more preferably a methyl group or a phenyl group, and even more preferably a methyl group.

From the viewpoint of further improving the durability of the treatment effect of the keratin substance or the fiber for a head decoration product, $R^{42}$ is preferably a hydroxy group, an alkyl group having 1 or more and 6 or less carbon atoms, or a phenyl group, more preferably a hydroxy group, an alkyl group having 1 or more and 3 or less carbon atoms, or a phenyl group, still more preferably a hydroxy group, a methyl group, or a phenyl group, and even more preferably a methyl group.

In the general formula (4), $R^{43}$ represents a hydrocarbon group having 1 or more and 6 or less carbon atoms.

The hydrocarbon group in $R^{43}$ is the same as in $R^{41}$, and is preferably an alkyl group having 1 or more and 6 or less carbon atoms or a phenyl group, more preferably an alkyl group having 1 or more and 3 or less carbon atoms or a phenyl group, still more preferably a methyl group or a phenyl group, and even more preferably a methyl group.

In the general formula (4), $n_E$ represents a degree of polymerization, and is a number of preferably 100 or more, more preferably 150 or more, still more preferably 250 or more, even more preferably 300 or more, even more preferably 320 or more, and even more preferably 350 or more, from the viewpoint of further improving the durability of the treatment effect of the keratin substance or the fiber for a head decoration product. On the other hand, from the viewpoint of further improving the durability of the treatment effect of the keratin substance or the fiber for a head decoration product, and from the viewpoint of availability, the degree of polymerization is preferably 4,300 or less, more preferably 4,200 or less, still more preferably 4,000 or less, even more preferably 3,800 or less, even more preferably 3,700 or less, and even more preferably 3,650 or less. Accordingly, $n_E$ of the general formula (4) is preferably 100 or more and 4,300 or less, more preferably 150 or more and 4,200 or less, still more preferably 250 or more and 4,200 or less, even more preferably 300 or more and 4,000 or less, even more preferably 320 or more and 3,800 or less, even more preferably 320 or more and 3,700 or less, and even more preferably 350 or more and 3,650 or less.

The viscosity at 25°C of the component (E) is preferably 120 $mm^2/s$ or more, more preferably 200 $mm^2/s$ or more, still more preferably 500 $mm^2/s$ or more, even more preferably 700 $mm^2/s$ or more, even more preferably 800 $mm^2/s$ or more, and even more preferably 1,000 $mm^2/s$ or more, from the viewpoint of further improving the durability of the treatment effect of the keratin substance or the fiber for a head decoration product. On the other hand, from the viewpoint of further improving the durability of the treatment effect of the keratin substance or the fiber for a head decoration product, and from the viewpoint of availability, the viscosity at 25°C of the component (E) is preferably 100,000,000 $mm^2/s$ or less, more preferably 80,000,000 $mm^2/s$ or less, still more preferably 50,000,000 $mm^2/s$ or less, even more preferably 40,000,000 $mm^2/s$ or less, even more preferably 35,000,000 $mm^2/s$ or less, even more preferably 30,000,000 $mm^2/s$ or less, and even more preferably 25,000,000 $mm^2/s$ or less.

Therefore, the viscosity at 25°C of the component (E) is preferably 120 $mm^2/s$ or more and 100,000,000 $mm^2/s$ or less, more preferably 200 $mm^2/s$ or more and 80,000,000 $mm^2/s$ or less, still more preferably 500 $mm^2/s$ or more and 80,000,000 $mm^2/s$ or less, even more preferably 700 $mm^2/s$ or more and 80,000,000 $mm^2/s$ or less, even more preferably 700 $mm^2/s$ or more and 50,000,000 $mm^2/s$ or less, even more preferably 800 $mm^2/s$ or more and 40,000,000 $mm^2/s$ or less, even more preferably 800 $mm^2/s$ or more and 35,000,000 $mm^2/s$ or less, even more preferably 800 $mm^2/s$ or more and 30,000,000 $mm^2/s$ or less,

and even more preferably 1,000 $mm^2/s$ or more and 25,000,000 $mm^2/s$ or less.

The viscosity is a value measured at 25°C based on JIS Z 8803:2011 "Methods for viscosity measurement of liquid", and can be measured by selecting an appropriate one from a capillary viscometer, a falling ball viscometer, a rotary viscometer, and a vibration viscometer. In addition, when the viscosity exceeds the common measurement range of a viscometer, it can be obtained from a diluted solution of the component (E) by the following method.

A toluene solution of the component (E) having a concentration of 1 g/100 mL is prepared, and the specific viscosity $\eta sp$ (25°C) is determined by the following equation (1). Next, the intrinsic viscosity $[\eta]$ is determined by substi-

tuting the relationship of Huggins shown in the following equation (2). Further, [η] is substituted into the A. Kolorlov equation shown in the following equation (3) to determine the molecular weight M. Finally, M is substituted into the A. J. Barry equation shown in the following equation (4) to determine the viscosity η of the component (B) (see, for example, Silicone Oil KF-96 Performance Test Result 4.2, published by Shin-Etsu Chemical Co., Ltd.).

$$\eta sp = (\eta/\eta 0) - 1 \quad (1)$$

(η0: viscosity of toluene, η: viscosity of solution)

$$\eta sp = [\eta] + K'[\eta]^2 \quad (2)$$

(K': Huggins constant, and the one described in Nakamuta, Nihon Kagakukai-shi, 77588 [1956] is used.)

$$[\eta] = 0.215 \times 10^{-4} M^{0.65} \quad (3)$$

$$\log\eta = 1.00 + 0.0123 M^{0.5} \quad (4)$$

Examples of a commercially available product of dimethylpolysiloxane used as the component (E) include KF-96-1,000cs (viscosity of 1,000 $mm^2/s$), KF-96H-10000cs (viscosity of 10,000 $mm^2/s$), KF-96H-100000cs (viscosity of 100,000 $mm^2/s$), KF-96H-300000cs (viscosity of 300,000 $mm^2/s$), KF-96H-500000cs (viscosity of 500,000 $mm^2/s$), X-21-5686 (viscosity of 3,000,000 $mm^2/s$), and X-25-9074 (viscosity of 30,000,000 $mm^2/s$) (all manufactured by Shin-Etsu Chemical Co., Ltd.), and Silsoft B3020 (viscosity of 20,000,000 $mm^2/s$) (manufactured by Momentive Performance Materials, Inc.).

Examples of a commercially available product of dimethiconol used as the component (E) include X-21-5613 (20% by mass - low viscosity dimethylpolysiloxane solution), X-21-5666 (30% by mass - cyclopentasiloxane solution), X-21-5847, and X-21-5849 (all manufactured by Shin-Etsu Chemical Co., Ltd.).

From the viewpoint of further improving the durability of the treatment effect of the keratin substance or the fiber for a head decoration product, the component (E) is preferably one or more selected from the group consisting of dimethylpolysiloxane, methylphenylpolysiloxane, and dimethiconol having a degree of polymerization within the above range, and more preferably dimethylpolysiloxane having a degree of polymerization within the above range.

When the first agent contains the component (E), the content of the component (E) in the first agent is preferably 0.1% by mass or more, more preferably 0.3% by mass or more, still more preferably 0.5% by mass or more, and even more preferably 1% by mass or more, and is preferably 30% by mass or less, more preferably 25% by mass or less, still more preferably 20% by mass or less, even more preferably 15% by mass or less, and even more preferably 10% by mass or less, from the viewpoint of further improving the durability of the treatment effect of the keratin substance or the fiber for a head decoration product. Thus, the content of the component (E) in the first agent is preferably 0.1% by mass or more and 30% by mass or less, more preferably 0.3% by mass or more and 25% by mass or less, still more preferably 0.5% by mass or more and 20% by mass or less, even more preferably 1% by mass or more and 15% by mass or less, and even more preferably 1% by mass or more and 10% by mass or less.

(Solvent)

The first agent preferably further contains a solvent from the viewpoint of dissolving or dispersing the component (A), the component (B), and other components, and from the viewpoint of adjusting the viscosity to a viscosity that is easy to apply to the keratin substance or the fiber for a head decoration product.

Examples of the solvent include alcohol-based solvents, ether-based solvents, ketone-based solvents, ester-based solvents, hydrocarbon-based solvents, and silicone-based solvents, and the solvent can be appropriately selected according to the formulation. From the viewpoint of ease of drying after application to the keratin substance or the fiber for a head decoration product, it is preferable to include a volatile solvent.

Among the volatile solvents, examples of the alcohol-based solvent include ethanol, 1-propanol, 2-propanol, 1-butanol, 2-butanol, and benzyl alcohol; examples of the ether-based solvent include diethyl ether and tetrahydrofuran; examples of the ketone-based solvent include acetone and methyl ethyl ketone; examples of the ester-based solvent include methyl acetate, ethyl acetate, butyl acetate, and isobutyl acetate; examples of the hydrocarbon-based solvent include light liquid isoparaffin (containing isoparaffin having 8 to 16 carbon atoms as a main component), pentane,

isopentane, hexane, isohexane, heptane, isoheptane, decane, isodecane, dodecane, isododecane, tridecane, isotridecane, tetradecane, and isotetradecane; and examples of the silicone-based solvent include cyclic silicone such as decamethylcyclopentasiloxane, dimethylpolysiloxane having a viscosity at 25°C of 4 $mm^2/s$ or less, and alkyl trimethicone such as methyl trimethicone. One or more of these solvents can be used.

From the viewpoint of the solubility of the component (A) and the component (B), and from the viewpoint of the ease of drying after the first agent is applied to the keratin substance or the fiber for a head decoration product, the first agent preferably contains one or more solvents selected from the group consisting of ethanol, 1-propanol, 2-propanol, dimethylpolysiloxane having a viscosity at 25°C of 4 $mm^2/s$ or less, methyltrimethicone, pentane, isopentane, hexane, isohexane, heptane, isoheptane, decane, isodecane, dodecane, isododecane, tridecane, isotridecane, tetradecane, isotetradecane, and light liquid isoparaffin, more preferably one or more solvents selected from the group consisting of dimethylpolysiloxane having a viscosity at 25°C of 4 $mm^2/s$ or less, methyltrimethicone, isodecane, isododecane, isotetradecane, and light liquid isoparaffin, and still more preferably one or more solvents selected from the group consisting of isodecane, isododecane, isotetradecane, and light liquid isoparaffin.

When the first agent contains a solvent, the content of the solvent in the first agent is preferably 40% by mass or more, more preferably 50% by mass or more, and still more preferably 60% by mass or more, and is preferably 99.94% by mass or less, and more preferably 98% by mass or less, from the viewpoint of adjusting the viscosity of the first agent so as to be easily applied to a keratin substance or a fiber for a head decoration product. Accordingly, the content of the solvent in the first agent is preferably 40% by mass or more and 99.94% by mass or less, more preferably 50% by mass or more and 98% by mass or less, and still more preferably 60% by mass or more and 98% by mass or less.

In addition to the above-described components, the first agent can contain components usually used in a cosmetic composition, for example, a functional powder other than a colorant, an oil agent other than the above-described components, an antioxidant, a perfume, a pigment, a dye, an antiseptic, a viscosity improver, a pH modifier, a blood circulation promoting agent, a cooling agent, an antiperspirant, a bactericide, a skin activating agent, a moisturizing agent, and a refreshing agent.

The first agent preferably has small amount of water from the viewpoint of film formability in the step (I) and improving the durability of the treatment effect of the keratin substance or the fiber for a head decoration product, from the viewpoint of improving the removability of the film by the step (II), and from the viewpoint of improving the quick-drying property and the feel of the keratin substance or the fiber for a head decoration product after the step (II). The content of water in the first agent is preferably 10% by mass or less, more preferably less than 5% by mass, and still more preferably less than 2% by mass.

The first agent preferably has a small content of a solid oil from the viewpoint of film formability in the step (I) and improving the durability of the treatment effect of the keratin substance or the fiber for a head decoration product, from the viewpoint of improving the removability of the film by the step (II), and from the viewpoint of improving the quick-drying property and the feel of the keratin substance or the fiber for a head decoration product after the step (II). The solid oil is an oil agent that is solid at 25°C, and examples thereof include paraffin-based waxes such as solid paraffin, polyolefin-based waxes such as polyethylene wax, and beeswax. The content of the solid oil in the first agent is preferably less than 50% by mass, more preferably less than 20% by mass, still more preferably less than 10% by mass, even more preferably less than 5% by mass, and even more preferably less than 1% by mass.

The first agent preferably has a small content of a nonvolatile liquid oil agent other than the components (B) and (E) from the viewpoint of film formability in the step (I) and improving the durability of the treatment effect of the keratin substance or the fiber for a head decoration product, from the viewpoint of improving the removability of the film by the step (II), and from the viewpoint of improving the quick-drying property and the feel of the keratin substance or the fiber for a head decoration product after the step (II). The nonvolatile liquid oil agent is an oil agent that has a boiling point higher than 260°C at atmospheric pressure and is liquid at 25°C, and examples thereof include nonvolatile hydrocarbon oils such as liquid isoparaffin; nonvolatile liquid silicones other than the components (B) and (E); triglycerides such as glyceryl trioctanoate, glyceryl tri-2-ethylhexanoate (triethylhexanoin), caprylic/capric triglyceride, avocado oil, olive oil, sesame seed oil, rice bran oil, safflower oil, soybean oil, corn oil, rapeseed oil, castor oil, cotton seed oil, and mink oil; fatty acids such as oleic acid and isostearic acid; ester oils such as isopropyl myristate, butyl myristate, isopropyl palmitate, ethyl oleate, ethyl linoleate, isopropyl linoleate, cetyl caprylate, hexyl laurate, decyl myristate, decyl oleate, oleyl oleate, isostearyl laurate, isotridecyl myristate, isocetyl myristate, isostearyl myristate, octyldodecyl myristate, octyl palmitate, isocetyl palmitate, isostearyl palmitate, isodecyl oleate, isopropyl isostearate, cetyl 2-ethylhexanoate, stearyl 2-ethylhexanoate, propylene glycol dicaprate, propylene glycol dioleate, isononyl isononanoate, diisopropyl sebacate, propylene glycol isostearate, 2-ethylhexyl paramethoxycinnamate, 2-ethoxyethyl paramethoxycinnamate, isopropyl paramethoxycinnamate/diisopropyl cinnamate ester mixture, methyl bis(trimethylsiloxy) silyl isopentyl trimethoxycinnamate, amyl paradimethylaminobenzoate, 2-ethylhexyl paradimethylaminobenzoate, ethylene glycol salicylate, 2-ethylhexyl salicylate, benzyl salicylate, homomenthyl salicylate, octocrylene, and dimethyl diethyl benzalmalonate; and branched or unsaturated higher alcohols such as 2-octyldodecanol, isostearyl alcohol, and oleyl alcohol. The content of the nonvolatile liquid oil agent in the first agent is preferably less than 50% by mass, more preferably less than 40%

by mass, still more preferably less than 30% by mass, even more preferably less than 20% by mass, and even more preferably less than 10% by mass.

The first agent preferably has a low content of volatile cyclic silicone such as decamethylcyclopentasiloxane from the viewpoint of the drying rate after application. This is because the volatile cyclic silicone has a slower volatilization time than other volatile oils and tends to take a longer time to dry after the application to the keratin substance or the fiber for a head decoration product. The content of the volatile cyclic silicone in the first agent is preferably less than 5% by mass, more preferably less than 2% by mass, still more preferably less than 1% by mass, even more preferably less than 0.5% by mass, even more preferably less than 0.1% by mass, and even more preferably 0% by mass in the first agent.

The first agent preferably has a small content of a polyhydric alcohol from the viewpoint of film formability in the step (I) and improving the durability of the treatment effect of the keratin substance or the fiber for a head decoration product, from the viewpoint of improving the removability of the film by the step (II), and from the viewpoint of improving the quick-drying property and the feel of the keratin substance or the fiber for a head decoration product after the step (II). Examples of the polyhydric alcohol include polyhydric alcohols having a boiling point of more than 260°C at normal pressure, and propylene glycol, and glycerol can be exemplified. The content thereof in the first agent is preferably less than 5% by mass, more preferably less than 2% by mass, still more preferably less than 1% by mass, even more preferably less than 0.5% by mass, and even more preferably less than 0.1% by mass.

The method for producing the first agent is not particularly limited, and the first agent can be produced according to a conventional method.

(Formulation)

The formulation of the first agent is not particularly limited, and can be made into a formulation such as a liquid, paste, cream, gel, foam, spray, and wax according to the product form. In addition, the first agent is preferably a non-aqueous composition from the viewpoint of film formability in the step (I) and improving the durability of the treatment effect of the keratin substance or the fiber for a head decoration product, from the viewpoint of improving the removability of the film by the step (II), and from the viewpoint of improving the quick-drying property and the feel of the keratin substance or the fiber for a head decoration product after the step (II). Here, the non-aqueous composition means a composition having a water content of less than 1% by mass, preferably less than 0.5% by mass, and more preferably less than 0.1% by mass.

The formulation of the first agent may be an aerosol type. Examples of the aerosol type preparation containing the first agent include a preparation in which the first agent, which is an aerosol stock solution, and a propellant are filled in an aerosol container. The composition of the first agent as an aerosol stock solution and the preferred range thereof are the same as described above.

Examples of the propellant used in the aerosol type preparation include liquefied petroleum gas (LPG) which is ethane, propane, normal butane, isobutane, isopentane and a mixture thereof; ethers such as dimethyl ether; and compressed gases such as nitrogen and carbon dioxide, and one or more of these can be used.

In the aerosol preparation, the amount ratio of the first agent, which is an aerosol stock solution, to the propellant varies depending on the type of the propellant, but from the viewpoint of aerosol performance, the mass ratio of the first agent to the propellant is preferably in the range of 1:0.01 to 1:10, and more preferably in the range of 1:0.05 to 1:7.5.

When the propellant is LPG, the mass ratio of the first agent to LPG is still more preferably 1:0.5 to 1:5; when the propellant is dimethyl ether, the mass ratio of the first agent to dimethyl ether is still more preferably 1:0.1 to 1:5; and when the propellant is carbon dioxide, the mass ratio of the first agent to carbon dioxide is still more preferably 1:0.1 to 1:0.5.

Examples of the aerosol container used for the aerosol type preparation include a known pressure-resistant container made of metal or plastic, and a double-structure container in which an inner bag is accommodated inside the pressure-resistant container. In the double-structure container, it is preferable that the inner bag is filled with the aerosol stock solution, and a propellant is filled between the pressure-resistant container and the inner bag.

As the type of the first agent, a cosmetic composition is preferable, and examples thereof include a skin cosmetic composition, a cosmetic composition for eyebrows or eyelashes, a hair cosmetic composition, and a nail cosmetic composition. The hair cosmetic composition can also be applied to fibers for head decoration products.

Examples of the skin cosmetic composition include various skin cosmetic compositions for make-up, foundation, skin care, and sunscreen.

Examples of the cosmetic composition for eyebrows or eyelashes include mascara, mascara base, mascara top coat, and eyebrow mascara.

Examples of the hair cosmetic composition include a conditioning agent composition, a treatment agent composition (including a leave-on type), a styling agent composition, a hair dye composition, and a hair growth agent composition.

Examples of the nail cosmetic composition include various nail cosmetic compositions such as a manicure, a

base coat for a nail, and a topcoat for a nail.

Among the above, from the viewpoint of forming a film, the first agent is preferably a hair cosmetic composition, more preferably a conditioning agent composition, a treatment agent composition, a styling agent composition, or a hair dye composition, and still more preferably a hair dye composition.

In addition, from the viewpoint of forming a film, the first agent is preferably a socalled leave-on preparation which is used without being washed off after being applied to keratin substances or fibers for head decoration products by application.

(Application Method of First Agent)

The keratin substance or the fiber for a head decoration product when the first agent is applied in the step (I) may be in a dry state or a wet state, but from the viewpoint of obtaining the effect of the present invention, it is preferable to apply the first agent to the keratin substance or the fiber for a head decoration product in a dry state. The method for applying the first agent can be appropriately selected depending on the formulation of the first agent, and examples thereof include coating, casting, and spraying.

From the viewpoint of uniform coatability, it is preferable that the first agent is applied to the surface of the keratin substance or the fiber for a head decoration product after being temporarily compatibilized or dispersed before being applied to the keratin substance or the fiber for a head decoration product. As a means for temporarily compatibilization or dispersion, a thermodynamic means such as heating, a physical means such as mechanically applying shear stress, or a chemical means such as adding a compatible solvent can be arbitrarily used. From the viewpoint of user convenience, it is preferable to uniformly compatibilize or disperse the first agent by physical means such as stirring and shaking.

In the step (I), the amount of the first agent applied to the keratin substance or the fiber for a head decoration product is not particularly limited, but when applied to the skin or the nail, it is usually in the range of 0.1 mg or more and 1000 mg or less per 1 $cm^2$ of the skin or the nail. When the first agent is applied to eyebrows, eyelashes, hair, or fibers for head decoration products, the amount of the first agent applied is usually in the range of 0.005 g or more and 1 g or less per 1 g of eyebrows, eyelashes, hair, or fibers for head decoration products.

(Drying Step)

In the step (I), from the viewpoint of forming a film by applying the first agent to the keratin substance or the fiber for a head decoration product, it is preferable to include a step of drying after applying the first agent. In addition, from the viewpoint of maintaining the treatment effect, it is preferable not to wash away the first agent after drying.

The drying time is not particularly limited as long as the film is substantially formed on the surface of the keratin substance or the fiber for a head decoration product after the application of the first agent, and is appropriately adjusted according to the application amount and the area of the application site, but is preferably 4 minutes or less, and more preferably 2 minutes or less.

The drying method after applying the first agent to the keratin substance or the fiber for a head decoration product may be natural drying. When the application object of the present invention is hair or a fiber for a head decoration product, from the viewpoint of rapidly forming a film, a step of applying the first agent to hair and then drying the hair using a device such as a hood, a hair dryer, or a straightening iron may be performed.

In a case where the device is used, from the viewpoint of suppressing heat damage to the hair or the fiber for a head decoration product, it is preferable that drying is performed by applying a temperature of 40°C or higher and 220°C or lower. Drying is more preferably performed by a hood or a hair dryer, and the drying temperature is preferably 40°C or higher and 110°C or lower, and more preferably 50°C or higher and 90°C or lower.

The drying time by the device is not particularly limited as long as the film is substantially formed on the surface of the hair or the fiber for a head decoration product, and is appropriately adjusted depending on the amount and quality of the hair or the fiber for a head decoration product, but can be performed in the range of, for example, 10 seconds to 120 minutes.

After drying, brushing may be performed in order to loosen the hair or the fiber for a head decoration product.

The keratin substance or the fiber for a head decoration product treated in the step (I) by the above method has high durability of the treatment effect and good feel after the treatment.

<Step (II)>

Step (II) is a step of applying a second agent to the keratin substance or the fiber for a head decoration product on which the film is formed in the step (I) to remove the film, and the second agent contains a component (C): an oil agent. Hereinafter, the components contained in the second agent used in step (II) will be described.

(Component (C): Oil Agent)

The second agent contains an oil agent as the component (C). The second agent contains the component (C), and thus the film formed using the first agent in the step (I) can be easily removed. In addition, the quick-drying property and the feel of the keratin substance or the fiber for a head decoration product after removal of the film are also improved.

The component (C) used in the second agent is preferably an oil agent which is in a liquid state at 25°C from the viewpoint of ease of application to a keratin substance or a fiber for a head decoration product. The viscosity of the component (C) at 25°C is preferably 300 mPa·s or less, more preferably 200 mPa·s or less, still more preferably 180 mPa·s or less, even more preferably 100 mPa·s or less, even more preferably 50 mPa·s or less, and even more preferably 40 mPa·s or less, from the viewpoint of the removability of the film by the step (II), and from the viewpoint of improving the quick-drying property and the feel of the keratin substance or the fiber for a head decoration product after the step (II). On the other hand, from the viewpoint of improving the coatability to the keratin substance or the fiber for a head decoration product, the viscosity is preferably 1 mPa·s or more.

The viscosity of the component (C) at 25°C can be measured by a rotary viscometer, and specifically, can be measured by the method described in Examples.

As the component (C), any of a volatile oil agent and a nonvolatile oil agent can be used.

Examples of the volatile oil agent include volatile solvents exemplified for the first agent. Examples of the volatile solvent include hydrocarbon-based solvents such as light liquid isoparaffin (containing isoparaffin having 8 to 16 carbon atoms as a main component), pentane, isopentane, hexane, isohexane, heptane, isoheptane, decane, isodecane, dodecane, isododecane, tridecane, isotridecane, tetradecane, and isotetradecane; silicone-based solvents such as cyclic silicone such as decamethylcyclopentasiloxane, dimethylpolysiloxane having a viscosity at 25°C of 4 $mm^2/s$ or less, and alkyl trimethicone such as methyl trimethicone; ester-based solvents such as methyl acetate, ethyl acetate, butyl acetate, and isobutyl acetate; alcohol-based solvents such as ethanol, 1-propanol, 2-propanol, 1-butanol, 2-butanol, and benzyl alcohol; ether-based solvents such as diethyl ether and tetrahydrofuran; and ketone-based solvents such as acetone and methyl ethyl ketone. One or more of these solvents can be used.

Among the above, one or more solvents selected from the group consisting of a hydrocarbon-based solvent and a silicone-based solvent are preferable, and one or more solvents selected from the group consisting of light liquid isoparaffin, isododecane, and dimethylpolysiloxane having a viscosity at 25°C of 4 $mm^2/s$ or less are more preferable, from the viewpoint of the removability of the film by the step (II) and from the viewpoint of improving the quick-drying property and the feel of the keratin substance or the fiber for a head decoration product after the step (II).

Examples of the nonvolatile oil agent include nonvolatile liquid solvents exemplified for the first agent. Examples the nonvolatile oil agent include nonvolatile hydrocarbon oils such as liquid isoparaffin; nonvolatile liquid silicones other than the components (B) and (E), such as dimethylpolysiloxane having a viscosity at 25°C of 5 $mm^2/s$ or more and 50 $mm^2/s$ or less; triglycerides such as glyceryl trioctanoate, glyceryl tri-2-ethylhexanoate (triethylhexanoin), caprylic/capric triglyceride, avocado oil, olive oil, sesame seed oil, rice bran oil, safflower oil, soybean oil, corn oil, rapeseed oil, castor oil, cotton seed oil, and mink oil; fatty acids such as oleic acid and isostearic acid; ester oils such as isopropyl myristate, butyl myristate, isopropyl palmitate, ethyl oleate, ethyl linoleate, isopropyl linoleate, cetyl caprylate, hexyl laurate, decyl myristate, decyl oleate, oleyl oleate, isostearyl laurate, isotridecyl myristate, isocetyl myristate, isostearyl myristate, octyldodecyl myristate, octyl palmitate, isocetyl palmitate, isostearyl palmitate, isodecyl oleate, isopropyl isostearate, cetyl 2-ethylhexanoate, stearyl 2-ethylhexanoate, propylene glycol dicaprate, propylene glycol dioleate, isononyl isononanoate, diisopropyl sebacate, propylene glycol isostearate, 2-ethylhexyl paramethoxycinnamate, 2-ethoxyethyl paramethoxycinnamate, isopropyl paramethoxycinnamate/diisopropyl cinnamate ester mixture, methyl bis(trimethylsiloxy) silyl isopentyl trimethoxycinnamate, amyl paradimethylaminobenzoate, 2-ethylhexyl paradimethylaminobenzoate, ethylene glycol salicylate, 2-ethylhexyl salicylate, benzyl salicylate, homomenthyl salicylate, octocrylene, and dimethyl diethyl benzalmalonate; and branched or unsaturated higher alcohols such as 2-octyldodecanol, isostearyl alcohol, and oleyl alcohol. One or more of these can be used.

Among them, one or more selected from the group consisting of nonvolatile hydrocarbon oils, nonvolatile liquid silicones, triglycerides, and ester oils are preferable, one or more selected from the group consisting of nonvolatile hydrocarbon oils, nonvolatile liquid silicones, and triglycerides are more preferable, and one or more selected from the group consisting of liquid isoparaffin, dimethylpolysiloxane having a viscosity at 25°C of 5 $mm^2/s$ or more and 50 $mm^2/s$ or less, and triethylhexanoin are still more preferable, from the viewpoint of the removability of the film by the step (II) and from the viewpoint of improving the quick-drying property and the feel of the keratin substance or the fiber for a head decoration product after the step (II).

From the viewpoint of the removability of the film by the step (II) and from the viewpoint of improving the quick-drying property and the feel of the keratin substance or the fiber for a head decoration product after the step (II), the component (C) is even more preferably one or more selected from the group consisting of light liquid isoparaffin, isododecane, dimethylpolysiloxane having a viscosity at 25°C of 50 $mm^2/s$ or less, liquid isoparaffin, and triethylhexanoin, and even more preferably one or more selected from the group consisting of light liquid isoparaffin, isododecane, dimethylpolysiloxane having a viscosity at 25°C of 50 $mm^2/s$ or less, and triethylhexanoin.

(Component (D): Surfactant)

The second agent preferably further contains a surfactant as a component (D) from the viewpoint of further improving the removability of the film by the step (II).

Examples of the surfactant include a cationic surfactant, an anionic surfactant, an amphoteric surfactant, and a nonionic surfactant, and one or more of these can be used.

<<Cationic Surfactant>>

Examples of the cationic surfactant include one or more selected from the group consisting of (i) alkyltrimethylammonium salts, (ii) alkoxyalkyltrimethylammonium salts, (iii) dialkyldimethylammonium salts, (iv) alkylamidoalkyltrimethylammonium salts, (v) alkyldimethylamines and salts thereof, (vi) alkoxyalkyldimethylamines and salts thereof, and (vii) alkylamidoalkyldimethylamines and salts thereof.

<<Anionic Surfactant>>

Examples of the anionic surfactant include one or more selected from the group consisting of alkyl benzene sulfonates, alkyl or alkenyl ether sulfates, alkyl or alkenyl sulfates, polyoxyethylene alkyl ether sulfates, alkyl sulfonates, α-olefin sulfonates, saturated or unsaturated fatty acid salts, alkyl or alkenyl ether carboxylates, α-sulfo fatty acid salts, N-acyl amino acids, phosphoric acid mono- or di-esters, and sulfosuccinic acid esters.

Examples of the counter ion of the anionic group in the anionic surfactant include alkali metal ions such as sodium ion and potassium ion; alkaline earth metal ions such as calcium ion and magnesium ion; ammonium ion; and alkanolammonium having 1 to 3 alkanol groups having 2 or 3 carbon atoms (for example, monoethanolammonium, diethanolammonium, triethanolammonium, triisopropanolammonium).

Among the above examples, as the anionic surfactant, one or more selected from the group consisting of alkyl sulfates, alkyl ether sulfates, and alkyl ether carboxylates are preferable, and alkyl ether sulfates are more preferable. Examples of the alkyl ether sulfate include polyoxyethylene alkyl ether sulfates such as sodium laureth sulfate and ammonium laureth sulfate, and examples of the alkyl ether carboxylate include polyoxyethylene alkyl ether acetates such as sodium laureth acetate.

<<Amphoteric Surfactant>>

Examples of the amphoteric surfactant include a betaine-type surfactant, an amine oxide-type surfactant, and an amino acid-type surfactant. Among these, a betaine-type surfactant is preferable.

Examples of the betaine-type surfactant include carboxybetaine-type such as alkyl dimethylaminoacetic acid betaine and fatty acid amide propyl betaine; sulfobetaine-type such as alkyl sulfobetaine and alkyl hydroxy sulfobetaine; imidazoline-based betaine-type; and phosphobetaine-type.

As the alkyl dimethylaminoacetic acid betaine, those having an alkyl group having 8 or more and 22 or less carbon atoms are preferable, and those having 10 or more and 20 or less carbon atoms are more preferable. Specific examples thereof include lauryl dimethylaminoacetic acid betaine and stearyl dimethylaminoacetic acid betaine.

As the fatty acid amide propyl betaine, those having an acyl group having 8 or more and 18 or less carbon atoms are preferable, and those having 10 or more and 16 or less carbon atoms are more preferable. Specific examples thereof include lauric acid amidopropyl betaine (lauramidopropyl betaine), palm kernel oil fatty acid amidopropyl betaine, and coconut oil fatty acid amidopropyl betaine (cocamidopropyl betaine).

Examples of the alkyl sulfobetaine include alkyl sulfobetaines having an alkyl group having preferably 8 or more and 22 or less carbon atoms, and more preferably 10 or more and 18 or less carbon atoms. Specific examples of the alkyl sulfobetaine include lauryl dimethyl sulfoethyl betaine, lauryl dimethyl sulfopropyl betaine, myristyl dimethyl sulfoethyl betaine, myristyl dimethyl sulfopropyl betaine, stearyl dimethyl sulfoethyl betaine, stearyl dimethyl sulfopropyl betaine, and coconut oil fatty acid dimethyl sulfopropyl betaine.

Examples of the alkyl hydroxy sulfobetaine include alkyl hydroxy sulfobetaines having an alkyl group having preferably 8 or more and 22 or less carbon atoms, more preferably 10 or more and 18 or less carbon atoms, and at least one hydroxy group. Specific examples of the alkyl hydroxy sulfobetaine include lauryl dimethyl sulfo(hydroxyethyl) betaine, lauryl dimethyl sulfo(hydroxypropyl) betaine [lauryl hydroxysulfobetaine], myristyl dimethyl sulfo(hydroxyethyl) betaine, myristyl dimethyl sulfo(hydroxypropyl) betaine, stearyl dimethyl sulfo(hydroxypropyl) betaine, bis-(2-hydroxyethyl) sulfoethyl betaine, and lauryl bis-(2-hydroxy-ethyl) sulfopropyl betaine. Among these, lauryl dimethyl sulfo(hydroxypropyl) betaine [lauryl hydroxysulfobetaine] represented by the following formula is preferable.

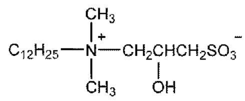

Examples of the imidazoline-based betaine type include 2-alkyl-N-carboxymethyl-N-hydroxyethylimidazolinium betaine, and specific examples thereof include sodium cocoamphoacetate.
Examples of the phosphobetaine type include lauryl hydroxyphosphobetaine.
Among the above, the amphoteric surfactant is more preferably one or more selected from the group consisting of carboxybetaine type, sulfobetaine type, and imidazoline-based betaine type, still more preferably one or more selected from the group consisting of alkyl dimethylaminoacetic acid betaine, fatty acid amide propyl betaine, alkyl sulfobetaine, alkyl hydroxy sulfobetaine, and 2-alkyl-N-carboxymethyl-N-hydroxyethyl imidazolinium betaine, even more preferably alkyl hydroxy sulfobetaine, and even more preferably lauryl dimethyl sulfobetaine (lauryl hydroxysulfobetaine).

<<Nonionic Surfactant>>

Examples of the nonionic surfactant include a polyoxyethylene alkyl ether, a polyoxyethylene alkenyl ether, a sucrose fatty acid ester, a polyoxyethylene fatty acid ester, a polyglyceryl alkyl ether, a fatty acid polyglyceryl, an alkyl glucoside, a polyoxyethylene alkylamine, a sorbitan fatty acid ester, a polyoxyethylene sorbitan fatty acid ester, a polyoxyethylene sorbitol fatty acid ester, and a polyoxyethylene hydrogenated castor oil, and one or more of these can be used.
Among the nonionic surfactants, the alkyl group in polyoxyethylene alkyl ether, polyglyceryl alkyl ether, alkyl glucoside, and polyoxyethylene alkylamine, the alkenyl group in polyoxyethylene alkenyl ether, and the fatty acid in sucrose fatty acid ester, polyoxyethylene fatty acid ester, fatty acid polyglyceryl, sorbitan fatty acid ester, polyoxyethylene sorbitan fatty acid ester, polyoxyethylene sorbitol fatty acid ester, and polyoxyethylene fatty acid ester have preferably 8 or more and 22 or less carbon atoms, more preferably 10 or more and 22 or less carbon atoms, and still more preferably 12 or more and 22 or less carbon atoms, from the viewpoint of further improving the removability of the film by the step (II).
Among the nonionic surfactants, the average addition mole number of ethylene oxide (hereinafter referred to as "EO average addition mole number") in polyoxyethylene alkyl ether, polyoxyethylene alkenyl ether, polyoxyethylene fatty acid ester, polyoxyethylene alkylamine, polyoxyethylene sorbitan fatty acid ester, polyoxyethylene sorbitol fatty acid ester, and polyoxyethylene hydrogenated castor oil is preferably 2 or more, more preferably 3 or more, and still more preferably 5 or more, and is preferably 150 or less, more preferably 80 or less, and still more preferably 60 or less, from the viewpoint of further improving the removability of the film by the step (II). The EO average addition mole number is a number average value.
Among the nonionic surfactants, from the viewpoint of further improving the removability of the film by the step (II), one or more surfactants selected from the group consisting of polyoxyethylene alkyl ether, polyoxyethylene alkenyl ether, sorbitan fatty acid ester, polyoxyethylene sorbitan fatty acid ester, and polyoxyethylene sorbitol fatty acid ester are preferable, one or more surfactants selected from the group consisting of polyoxyethylene sorbitan fatty acid ester and polyoxyethylene sorbitol fatty acid ester are more preferable, and polyoxyethylene sorbitol fatty acid ester is still more preferable. The number of carbon atoms of the alkyl group, the alkenyl group, or the fatty acid is preferably 8 or more and 22 or less, more preferably 10 or more and 22 or less, and still more preferably 12 or more and 22 or less.
Specific examples of the polyoxyethylene alkyl ether include polyoxyethylene lauryl ether, polyoxyethylene myristyl ether, polyoxyethylene cetyl ether, polyoxyethylene stearyl ether, and polyoxyethylene behenyl ether.
Specific examples of the polyoxyethylene alkenyl ether include polyoxyethylene oleyl ether.
Specific examples of the sorbitan fatty acid ester include sorbitan monolaurate and sorbitan monooleate.
Specific examples of the polyoxyethylene sorbitan fatty acid ester include polyoxyethylene sorbitan monolaurate, polyoxyethylene sorbitan monooleate, and polyoxyethylene coconut oil fatty acid sorbitan.
Specific examples of the polyoxyethylene sorbitol fatty acid ester include polyoxyethylene sorbitol mono-, di-, tri-, or tetra-lauric acid, polyoxyethylene sorbitol mono-, di-, tri-, or tetra-oleic acid, and polyoxyethylene sorbitol mono-, di-, tri-, or tetra-stearic acid.
As the component (D), one or more can be used either singly or as combined.
Among the above, from the viewpoint of the removability of the film by the step (II) and from the viewpoint of improving the quick-drying property and the feel of the keratin substance or the fiber for a head decoration product after the step (II), the component (D) is preferably one or more selected from the group consisting of an anionic surfactant, an amphoteric surfactant, and a nonionic surfactant, more preferably one or more selected from the group consisting of polyoxyethylene alkyl ether sulfate, alkyl hydroxysulfobetaine, and polyoxyethylene sorbitol fatty acid ester, and still more preferably one or more selected from the group consisting of sodium laureth sulfate, lauryl hydroxysulfobetaine, and polyoxyethylene sorbitol mono-, di-, tri-, or tetra-oleic acid.

(Content)

The content of the component (C) in the second agent is preferably 20% by mass or more, more preferably 30% by mass or more, still more preferably 40% by mass or more, even more preferably 50% by mass or more, even more preferably 70% by mass or more, even more preferably 80% by mass or more, and even more preferably 90% by mass or more, and is 100% by mass or less, from the viewpoint of the removability of the film by the step (II) and from the viewpoint of improving the quick-drying property and the feel of the keratin substance or the fiber for a head decoration product after the step (II).
When the second agent contains the component (D), the content of the component (D) in the second agent is preferably 0.1% by mass or more, more preferably 0.5% by mass or more, still more preferably 1% by mass or more, even more preferably 5% by mass or more, and even more preferably 10% by mass or more from the viewpoint of further improving the removability of the film by the step (II), and is preferably 70% by mass or less, more preferably 60% by mass or less, and still more preferably 50% by mass or less from the viewpoint of the removability of the film by the step (II) and from the viewpoint of improving the quick-drying property and the feel of the keratin substance or the fiber for a head decoration product after the step (II). Therefore, the content of the component (D) in the second agent is preferably 0.1% by mass or more and 70% by mass or less, more preferably 0.5% by mass or more and 60% by mass or less, still more preferably 1% by mass or more and 50% by mass or less, even more preferably 5% by mass or more and 50% by mass or less, and even more preferably 10% by mass or more and 50% by mass or less.
The total content of the component (C) and the component (D) in the second agent is preferably 30% by mass or more, more preferably 50% by mass or more, still more preferably 60% by mass or more, even more preferably 70% by mass or more, even more preferably 80% by mass or more, even more preferably 90% by mass or more, even more preferably 95 % by mass or more, and even more preferably 98% by mass or more, and is 100% by mass or less, from the viewpoint of the removability of the film by the step (II) and from the viewpoint of improving the quick-drying property and the feel of the keratin substance or the fiber for a head decoration product after the step (II).
In addition, the mass ratio of the content of the component (D) to the content of the component (C) [(D)/(C)] in the second agent is preferably 0.005 or more, and more preferably 0.010 or more, and is preferably 10 or less, more preferably 5 or less, still more preferably 3 or less, even more preferably 2 or less, even more preferably 1.5 or less, and even more preferably 1.2 or less, from the viewpoint of the removability of the film in the step (II) and from the viewpoint of improving the quick-drying property and the feel of the keratin substance or the fiber for a head decoration product after the step (II). Thus, the mass ratio of the content of the component (D) to the content of the component (C) [(D)/(C)] in the second agent is preferably 0.005 or more and 10 or less, more preferably 0.010 or more and 5 or less, still more preferably 0.010 or more and 3 or less, even more preferably 0.010 or more and 2 or less, even more preferably 0.010 or more and 1.5 or less, and even more preferably 0.010 or more and 1.2 or less.
In addition to the components (C) and (D), the second agent may appropriately contain a polyhydric alcohol, a colorant, an antioxidant, a perfume, an antiseptic, a viscosity improver, a pH modifier, a bactericide, a moisturizing agent, and a refreshing agent.

(Formulation)

The formulation of the second agent is not particularly limited, and can be made into a formulation such as a liquid, paste, cream, gel, foam, spray, and wax according to the product form. In addition, the second agent is preferably a non-aqueous system from the viewpoint of the removability of the film by the step (II) and from the viewpoint of improving the quick-drying property and the feel of the keratin substance or the fiber for a head decoration product after the step (II). That is, the content of water in the second agent is less than 1% by mass, preferably less than 0.5% by mass, and more preferably less than 0.1% by mass. Similarly to the first agent, the formulation of the second agent may also be an aerosol type. Examples of the aerosol type preparation containing the second agent include a preparation in which the second agent, which is an aerosol stock solution, and a propellant are filled in an aerosol container, and suitable embodiments thereof are the same as those described for the first agent.

(Application Method of Second Agent)

The keratin substance or the fiber for a head decoration product when the second agent is applied in the step (II) may be in a dry state or a wet state, but from the viewpoint of obtaining the effect of the present invention, it is preferable to apply the second agent to the keratin substance or the fiber for a head decoration product in a dry state. The method for applying the second agent can be appropriately selected depending on the formulation of the second agent, and examples thereof include coating, casting, and spraying.
In the step (II), the amount of the second agent applied to the keratin substance or the fiber for a head decoration product is not particularly limited, but when applied to the skin or the nail, it is usually preferably in the range of 0.0005

g or more and 5 g or less, more preferably in the range of 0.001 g or more and 3 g or less per 1 $cm^2$ of the skin or the nail. When the second agent is applied to eyebrows, eyelashes, hair, or fibers for head decoration products, the amount of the second agent applied is usually in the range of 0.005 g or more and 10 g or less, more preferably 0.01 g or more and 5 g or less per 1 g of eyebrows, eyelashes, hair, or fibers for head decoration products.

In the step (II), from the viewpoint of the removability of the film by the step (II) and from the viewpoint of improving the quick-drying property and the feel of the keratin substance or the fiber for a head decoration product after the step (II), it is more preferable to apply the second agent and the detergent to the keratin substance or the fiber for a head decoration product after the film is formed by the step (I).

The detergent may be a detergent other than the second agent used in the step (I), and may be a commercially available detergent for keratin substances. Examples of the commercially available detergent include a shampoo, and a cleansing agent for skin, eyelashes, eyebrows, or nails.

The detergent is preferably a detergent containing water as a main component, and more preferably a detergent containing a surfactant and water and containing water as a main component. Examples of the surfactant include various surfactants exemplified as the component (D) in the second agent.

The content of the surfactant in the detergent is preferably 0.01% by mass or more and 50% by mass or less, more preferably 0.1% by mass or more and 40% by mass or less, and still more preferably 1% by mass or more and 30% by mass or less from the viewpoint of improving the quick-drying property and the feel of the keratin substance or the fiber for a head decoration product after the step (II). In addition, the content of water in the detergent is preferably 50% by mass or more, more preferably 60% by mass or more, and still more preferably 70% by mass or more, and is preferably 99.99% by mass or less, more preferably 99.9% by mass or less, and still more preferably 99% by mass or less, from the viewpoint of improving the quick-drying property and the feel of the keratin substance or the fiber for a head decoration product after the step (II).

When the application object of the present invention is hair or fibers for hair decoration products, examples of the detergent used in the step (II) include shampoos containing at least an anionic surfactant and water. Examples of the anionic surfactant contained in the shampoo include the anionic surfactants exemplified as the component (D) in the second agent.

The content of the anionic surfactant in the shampoo can be selected in the range of usually 0.01% by mass or more and 40% by mass or less, preferably 0.1% by mass or more and 30% by mass or less, and more preferably 1% by mass or more and 20% by mass or less, and the content of water in the shampoo can be selected in the range of usually 60% by mass or more and 99.99% by mass or less, preferably 70% by mass or more and 99.9% by mass or less, and more preferably 70% by mass or more and 99% by mass or less.

When a detergent is used in the step (II), the detergent may be applied to the keratin substance or the fiber for a head decoration product at any timing before application of the second agent, simultaneously with application of the second agent, or after application of the second agent. From the viewpoint of the removability of the film by the step (II) and from the viewpoint of ease of implementation, it is preferable to apply a detergent after application of the second agent. Specifically, it is preferable to first apply the second agent to the keratin substance or the fiber for a head decoration product to spread the second agent to the keratin substance or the fiber for a head decoration product, and then apply the detergent to spread without washing away the second agent.

The amount of the detergent applied to the keratin substance or the fiber for a head decoration product in the step (II) is not particularly limited, but when applied to the skin or the nail, the amount is usually preferably in the range of 0.0005 g or more and 5 g or less, more preferably in the range of 0.001 g or more and 3 g or less per 1 $cm^2$ of the skin or the nail. When the detergent is applied to eyebrows, eyelashes, or hair, the amount of the detergent applied is usually in the range of 0.005 g or more and 10 g or less, more preferably 0.01 g or more and 5 g or less per 1 g of eyebrows, eyelashes, or hair.

In addition, in a case where a detergent is used in the step (II), the amount of the detergent with respect to the application amount of the second agent (detergent/second agent) is preferably in the range of 0.01 or more and 100 or less, and more preferably in the range of 0.1 or more and 10 or less as a mass ratio from the viewpoint of the removability of the film.

In the step (II), it is preferable that after the second agent (when a detergent is used, the second agent and the detergent) is applied to the keratin substance or the fiber for a head decoration product, the second agent (and the detergent) is allowed to spread, and then the second agent (and the detergent) is washed away with water. This makes it possible to easily remove the film formed in the step (I). The time for washing away with water is preferably 30 seconds or more and 5 minutes or less from the viewpoint of the removability of the film and the working efficiency.

After the film removal by the step (II), a step of drying the keratin substance or the fiber for a head decoration product can be performed in the same manner as in the step (I).

The keratin substance or the fiber for a head decoration product after the film removal by the step (II) has a quick-drying property and a good feel.

[Kit for Treating Keratin Substance or Fiber for Head Decoration Product]

The present invention further provides a kit for treating a keratin substance or a fiber for a head decoration product, including: a first agent containing a component (A): a silicone film-forming agent that is solid at 25°C, and a component (B): an organopolysiloxane other than the component (A), which has a cationic group, and a second agent containing a component (C): an oil agent.

The first agent, the second agent and preferred embodiments thereof are the same as described above.

The kit of the present invention may be provided with at least the first agent and the second agent, and may further be provided with a preparation that does not correspond to either the first agent or the second agent.

The first agent in the kit of the present invention may be composed of two or more compositions. These compositions are mixed before use to prepare the first agent, which can be applied to a keratin substance or a fiber for a head decoration product.

Regarding the above-mentioned embodiments, the present invention discloses the following.

<1> A method for treating a keratin substance or a fiber for a head decoration product, the method including, in the following order,

a step (I) of applying a first agent to the keratin substance or the fiber for a head decoration product to form a film, and
a step (II) of applying a second agent to the keratin substance or the fiber for a head decoration product on which the film has been formed to remove the film,
wherein the first agent contains a component (A): a silicone film-forming agent that is solid at 25°C, and a component (B): an organopolysiloxane other than the component (A), which has a cationic group, and the second agent contains a component (C): an oil agent.

<2> The method according to <1>, in which the keratin substance is preferably one or more selected from the group consisting of skin, eyebrows, eyelashes, hair, and nails, and more preferably hair.
<3> The method according to <1> or <2>, in which the head decoration product is one or more selected from the group consisting of a hair wig, a wig, weaving, hair extension, a blade hair, a hair accessory, and a doll hair.
<4> The method according to any one of <1> to <3>, in which the keratin substance or the fiber for a head decoration product is preferably one or more selected from the group consisting of skin, eyebrows, eyelashes, hair, nails, and fibers for head decoration products, more preferably one or more selected from the group consisting of hair and fibers for head decoration products, and still more preferably hair.
<5> The method according to any one of <1> to <4>, in which the component (A) is one or more selected from the group consisting of the following components (A1) and (A2): component (A1): a silicone resin represented by an average formula $(R^1)_mSiO_{(4-m)/2}$ (in which, $R^1$ represents a hydrocarbon group having 1 or more and 12 or less carbon atoms which may be fluorine-substituted or a hydroxy group; a plurality of $R^1$'s may be the same as or different from each other; and m is an average number which is more than 0 and less than 3), the silicone resin containing one or more units selected from the group consisting of a T unit represented by $R^1SiO_{3/2}$ and a Q unit represented by $SiO_{4/2}$; and
component (A2): a silicone polymer containing a polysiloxane moiety and a moiety formed of a non-silicone organic chain.
<6> The method according to <5>, in which the component (A1) includes one or more selected from the group consisting of the following component (A1-1) and component (A1-2):

(A1-1) a silicone resin represented by the above average formula, containing a T unit represented by $R^1SiO_{3/2}$ and substantially not containing a Q unit represented by $SiO_{4/2}$, and
(A1-2) a silicone resin represented by the above average formula, and containing a Q unit represented by $SiO_{4/2}$ and an M unit represented by $(R^1)_3SiO_{1/2}$.

<7> The method according to <5> or <6>, in which the component (A2) preferably includes one or more selected from the group consisting of the following components (A2-1) to (A2-4), and more preferably includes (A2-1):

(A2-1) An acryl silicone polymer;
(A2-2) A silicone-modified alicyclic structure-containing polymer;
(A2-3) A silicone-modified pullulan;
(A2-4) A polyurea/urethane silicone.

<8> The method according to <6> or <7>, in which the component (A1-1) is preferably polysilsesquioxanes, more preferably one or more selected from the group consisting of polymethylsilsesquioxane, polypropylsilsesquioxane, polyphenylsilsesquioxane, polymethylphenylsilsesquioxane, and fluorine-modified alkyldimethylpolysilsesquioxane, still more preferably one or more selected from the group consisting of polymethylsilsesquioxane and polypropylsilsesquioxane, and even more preferably polymethylsilsesquioxane.

<9> The method according to <6> or <7>, in which the component (A1-2) is preferably one or more selected from the group consisting of trimethylsiloxysilicate, trifluoropropyldimethyltrimethylsiloxysilicate and (trimethylsiloxysilicate/dimethiconol) crosspolymer, more preferably one or more selected from the group consisting of trimethylsiloxysilicate, and trifluoropropyldimethyltrimethylsiloxysilicate, and still more preferably trimethylsiloxysilicate.

<10> The method according to any one of <7> to <9>, in which the acryl silicone polymer of the component (A2-1) is one or more selected from the group consisting of an acrylic polymer having a carbosiloxane dendrimer structure in the side chain, an acryl-silicone graft copolymer, and a graft-type copolymer or alternate block-type copolymer where a structural unit of a polysiloxane group and a structural unit of a polymer of an unsaturated monomer via a sulfide bond, preferably one or more selected from the group consisting of an acrylic polymer having a carbosiloxane dendrimer structure in the side chain and an acryl-silicone graft copolymer, more preferably one or more selected from the group consisting of (acrylates/polytrimethylsiloxymethacrylate) copolymer and (acrylates/dimethicone) copolymer, and still more preferably (acrylates/dimethicone) copolymer.

<11> The method according to any one of <6> to <10>, in which the component (A) preferably includes one or more selected from the group consisting of the component (A1), the component (A2-1), and the component (A2-2), more preferably includes one or more selected from the group consisting of the component (A1) and the component (A2-1), still more preferably includes one or more selected from the group consisting of the component (A1-2) and the component (A2-1), and even more preferably includes the component (A1-2).

<12> The method according to any one of <5> to <11>, in which the component (A) contains both the component (A1) and the component (A2).

<13> The method according to <12>, in which the content of the component (A1) with respect to the total content of the component (A1) and the component (A2) is preferably 0.50 or more and 0.90 or less, more preferably 0.50 or more and 0.85 or less, and even more preferably 0.60 or more and 0.80 or less in terms of a mass ratio [(A1)/ f (A1) + (A2)}].

<14> The method according to any one of <1> to <13>, in which the component (A) contains one or more selected from the group consisting of trimethylsiloxysilicate, phenylpropyldimethylsiloxysilicate, trifluoropropyldimethyltrimethylsiloxysilicate, (trimethylsiloxysilicate/dimethiconol) crosspolymer, polymethylsilsesquioxane, polypropylsilsesquioxane, (acrylates/polytrimethylsiloxymethacrylate) copolymer, (acrylates/dimethicone) copolymer, and (norbornene/tris(trimethylsiloxy)silylnorbomene) copolymer, preferably contains one or more selected from the group consisting of trimethylsiloxysilicate, phenylpropyldimethylsiloxysilicate, trifluoropropyldimethyltrimethylsiloxysilicate, (trimethylsiloxysilicate/dimethiconol) crosspolymer, polymethylsilsesquioxane, polypropylsilsesquioxane, (acrylates/polytrimethylsiloxymethacrylate) copolymer, and (acrylates/dimethicone) copolymer, more preferably contains one or more selected from the group consisting of trimethylsiloxysilicate and (acrylates/dimethicone) copolymer, still more preferably contains trimethylsiloxysilicate, and even more preferably is trimethylsiloxysilicate.

<15> The method according to any one of <1> to <14>, in which the cationic group contained in the component (B) is a cationic group or a group which can be ionized to become a cationic group, preferably one or more selected from the group consisting of a primary amino group, a secondary amino group, a tertiary amino group, and a quaternary ammonium group, and more preferably one or more selected from the group consisting of a primary amino group, a secondary amino group, and a tertiary amino group.

<16> The method according to any one of <1> to <15>, in which the component (B) is one or more selected from the group consisting of an amino-modified silicone (B1) and an aminopolyether-modified silicone (B2).

<17> The method according to <16>, in which the amino-modified silicone (B1) is a silicone other than the component (A), which has an amino group and does not have a polyether moiety, and is preferably a silicone represented by the following general formula (1):

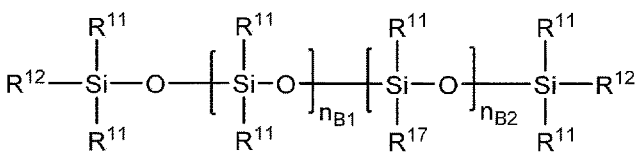

(1)

in which, each $R^{11}$ independently represents a hydrocarbon group having 1 or more and 6 or less carbon atoms, each $R^{12}$ independently represents a hydroxy group, an alkoxy group having 1 or more and 6 or less carbon atoms, or a hydrocarbon group having 1 or more and 22 or less carbon atoms, $R^{17}$ represents a primary to tertiary amino

group-containing group, $n_{B1}$ represents a number of 0 or more, $n_{B2}$ represents a number of 1 or more, $n_{B1}+n_{B2}$ represents a degree of polymerization, and $n_{B2}$'s $R^{17}$s may be the same as or different from each other.

<18> The method according to <16> or <17>, in which the aminopolyether-modified silicone (B2) is a silicone other than the component (A), which has an amino group and a polyether moiety, and is preferably one or more selected from the group consisting of an aminopolyether-modified silicone (B2-1) having a repeating unit represented by the following general formula (2) and an aminopolyether-modified silicone (B2-2) having a repeating unit represented by the following general formula (3):

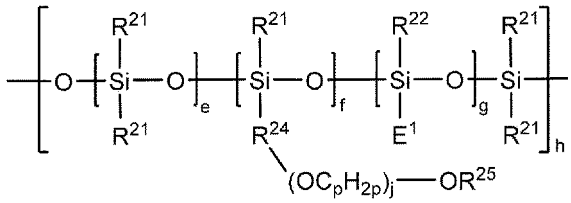

(2)

in the formula (2), $R^{21}$ independently represents a monovalent hydrocarbon group having 1 or more and 6 or less carbon atoms, $R^{22}$ represents any of $R^{21}$ or $E^1$, $E^1$ represents a monovalent group represented by $-R^{23}-Z^1$ (where $R^{23}$ represents a divalent hydrocarbon group having 1 or more and 6 or less carbon atoms, and $Z^1$ represents a primary to tertiary amino group-containing group), $R^{24}$ represents a divalent hydrocarbon group having 1 or more and 6 or less carbon atoms, $R^{25}$ represents a hydrogen atom or a monovalent hydrocarbon group having 1 or more and 4 or less carbon atoms, e is a number of 1 or more and 50 or less, f is a number of 1 or more and 50 or less, g is a number of 1 or more and 50 or less, h is a number of 1 or more, j is a number of 2 or more and 100 or less, p is a number of 2 or more and 10 or less. The bonding order of the parenthesized structural units is not limited, and the bonding form thereof may be in a block form or a random form. j's $(OC_pH_{2p})$s can be the same or different, and plural $R^{21}$'s, $R^{22}$'s, $R^{24}$'s, $R^{25}$'s and $E^1$'s can be the same or different;

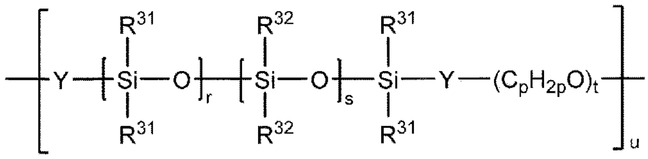

(3)

in the formula (3), $R^{31}$ represents a monovalent hydrocarbon group having 1 or more and 6 or less carbon atoms, $R^{32}$ represents any of $R^{31}$ or $E^2$, $E^2$ represents a monovalent group represented by $-R^{33}-Z^2$ (where $R^{33}$ represents a single bond, or a divalent hydrocarbon group having 1 or more and 20 or less carbon atoms, and $Z^2$ represents a primary to tertiary amino group-containing group), Y represents a single bond, or a divalent group having 1 or more and 12 or less carbon atoms. In the case where all $R^{32}$'s are $R^{31}$'s, at least one Y is an amino group-containing divalent group. In the case where all Y's are divalent groups not containing an amino group, at least one $R^{32}$ is $E^2$, r is a number of 1 or more, s is a number of 1 or more, t is a number of 2 or more and 100 or less, and u is a number of 1 or more, p is the same as above, and is a number of 2 or more and 10 or less. The bonding order of the parenthesized structural units is not limited, and the bonding form thereof may be in a block form or a random form. t's $(C_pH_{2p}O)$s can be the same or different, and plural $R^{31}$'s, $R^{32}$'s, $E^2$'s and Y's can be the same or different.

<19> The method according to <18>, in which the component (B2-1) is a compound represented by the following general formula (2-1):

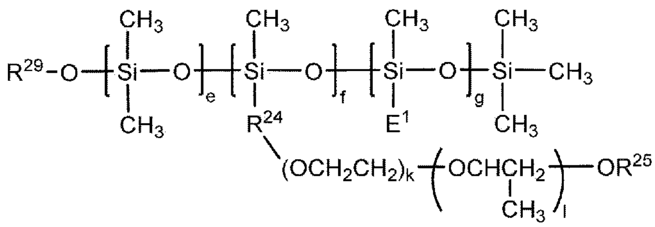

(2-1)

in which, $E^1$, $R^{24}$, $R^{25}$, e, f, and g are the same as above, $R^{29}$ represents a monovalent hydrocarbon group having 1 or more and 4 or less carbon atoms, or a trimethylsilyl group, k is a number of 1 or more and 50 or less, l is a

number of 0 or more and 50 or less, and is preferably a number of 1 or more and 50 or less.

<20> The method according to <18> or <19>, in which the component (B2-2) is one or more selected from the group consisting of an aminopolyether-modified silicone having a structure represented by the following general formula (3-1) and an aminopolyether-modified silicone having a structure represented by the following general formula (3-2), preferably one or more selected from the group consisting of an aminopolyether-modified silicone composed of a structure represented by the following general formula (3-1) and an aminopolyether-modified silicone composed of a structure represented by the following general formula (3-2), and more preferably an aminopolyether-modified silicone composed of a structure represented by the following general formula (3-1):

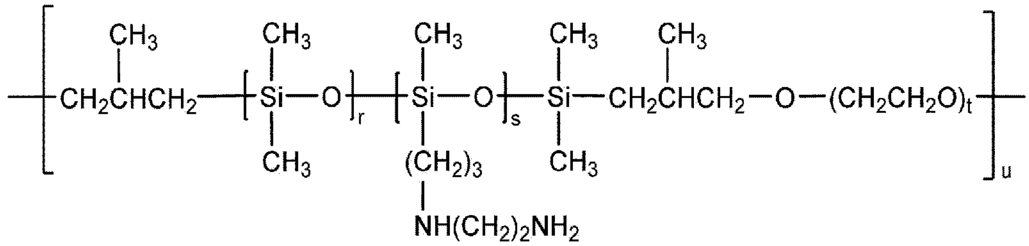
(3-1)

in the formula (3-1), r, s, t and u are the same as above,

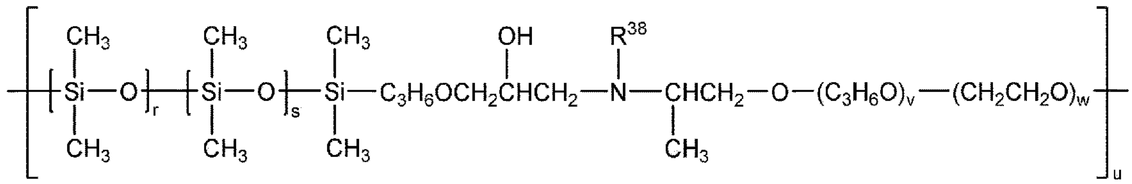
(3-2)

in the formula (3-2), $R^{38}$, r, s and u are the same as above, v is a number of 0 or more and 50 or less, and preferably a number of 2 or more and 50 or less. w is a number of 2 or more and 100 or less. v + w is a number of 2 or more and 100 or less, preferably a number of 4 or more and 80 or less, and more preferably a number of 10 or more and 50 or less.

<21> The method according to any one of <17> to <20>, in which the component (B) is preferably one or more selected from the group consisting of an amino-modified silicone represented by the general formula (1) and an aminopolyether-modified silicone (B2-2) having a repeating unit represented by the general formula (3), more preferably one or more selected from the group consisting of an amino-modified silicone represented by the general formula (1) and an aminopolyether-modified silicone of a structure represented by the general formula (3-1), and still more preferably an aminopolyether-modified silicone of a structure represented by the general formula (3-1), or the component (B) preferably includes one or more selected from the group consisting of bis-cetearyl amodimethicone and an aminopolyether-modified silicone (B2), more preferably includes one or more selected from the group consisting of bis-cetearyl amodimethicone and an aminopolyether-modified silicone (B2-2) having a repeating unit represented by the general formula (3), still more preferably includes one or more selected from the group consisting of bis-cetearyl amodimethicone and an aminopolyether-modified silicone composed of a structure represented by the general formula (3-1), and even more preferably is one or more selected from the group consisting of bis-cetearyl amodimethicone and an aminopolyether-modified silicone composed of a structure represented by the general formula (3-1).

<22> The method according to any one of <1> to <21>, in which the content of the component (A) in the first agent is preferably 0.05% by mass or more and 30% by mass or less, more preferably 0.1% by mass or more and 25% by mass or less, still more preferably 0.5% by mass or more and 25% by mass or less, even more preferably 1% by mass or more and 25% by mass or less, even more preferably 2% by mass or more and 25% by mass or less, even more preferably 3% by mass or more and 23% by mass or less, and even more preferably 5% by mass or more and 23% by mass or less.

<23> The method according to any one of <1> to <22>, in which the content of the component (B) in the first agent is preferably 0.01% by mass or more and 30% by mass or less, more preferably 0.02% by mass or more and 25% by mass or less, still more preferably 0.03% by mass or more and 20% by mass or less, even more preferably 0.03% by mass or more and 15% by mass or less, even more preferably 0.05% by mass or more and 10% by mass or less, even more preferably 0.05% by mass or more and 5% by mass or less, even more preferably 0.05% by mass or more and 3% by mass or less, and even more preferably 0.05% by mass or more and 2% by mass or less.

<24> The method according to any one of <1> to <23>, in which the total content of the component (A) and the

component (B) in the first agent is preferably 0.06% by mass or more and 50% by mass or less, more preferably 0.1% by mass or more and 45% by mass or less, still more preferably 0.5% by mass or more and 40% by mass or less, even more preferably 1% by mass or more and 30% by mass or less, even more preferably 2% by mass or more and 25% by mass or less, and even more preferably 5% by mass or more and 25% by mass or less.

<25> The method according to any one of <1> to <24>, in which the mass ratio of the content of the component (B) to the content of the component (A) [(B)/(A)] in the first agent is preferably 0.005 or more and 50 or less, more preferably 0.010 or more and 50 or less, still more preferably 0.010 or more and 30 or less, even more preferably 0.010 or more and 20 or less, even more preferably 0.010 or more and 10 or less, even more preferably 0.010 or more and 5 or less, even more preferably 0.010 or more and 3 or less, even more preferably 0.010 or more and 1 or less, and even more preferably 0.010 or more and 0.8 or less.

<26> The method according to any one of <1> to <25>, in which the first agent further contains a colorant, preferably a pigment or a dye, and more preferably a pigment.

<27> The method according to <26>, in which the content of the colorant in the first agent is preferably 0.01% by mass or more and 50% by mass or less, more preferably 0.05% by mass or more and 30% by mass or less, and still more preferably 0.1% by mass or more and 30% by mass or less.

<28> The method according to any one of <1> to <27>, in which the first agent further contains, as a component (E), an organopolysiloxane other than the component (A) and the component (B).

<29> The method according to <28>, in which the degree of polymerization of the component (E) is preferably 100 or more and 4,300 or less, more preferably 150 or more and 4,200 or less, still more preferably 250 or more and 4,200 or less, even more preferably 300 or more and 4,000 or less, even more preferably 320 or more and 3,800 or less, even more preferably 320 or more and 3,700 or less, and even more preferably 350 or more and 3,650 or less.

<30> The method according to <28> or <29>, in which the component (E) is an organopolysiloxane represented by the following general formula (4):

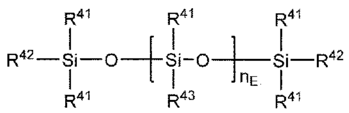 (4)

in which each $R^{41}$ independently represents a hydrocarbon group having 1 or more and 6 or less carbon atoms, each $R^{42}$ independently represents a hydroxy group, an alkoxy group having 1 or more and 6 or less carbon atoms, or a hydrocarbon group having 1 or more and 6 or less carbon atoms, $R^{43}$ represents a hydrocarbon group having 1 or more and 6 or less carbon atoms, $n_E$ represents a degree of polymerization, and $n_E$ is a number of 100 or more, and $n_E$'s $R^{43}$s may be the same or different from each other.

<31> The method according to any one of <28> to <30>, in which the viscosity at 25°C of the component (E) is preferably 120 $mm^2/s$ or more and 100,000,000 $mm^2/s$ or less, more preferably 200 $mm^2/s$ or more and 80,000,000 $mm^2/s$ or less, still more preferably 500 $mm^2/s$ or more and 80,000,000 $mm^2/s$ or less, even more preferably 700 $mm^2/s$ or more and 80,000,000 $mm^2/s$ or less, even more preferably 700 $mm^2/s$ or more and 50,000,000 $mm^2/s$ or less, even more preferably 800 $mm^2/s$ or more and 40,000,000 $mm^2/s$ or less, even more preferably 800 $mm^2/s$ or more and 35,000,000 $mm^2/s$ or less, even more preferably 800 $mm^2/s$ or more and 30,000,000 $mm^2/s$ or less, and even more preferably 1,000 $mm^2/s$ or more and 25,000,000 $mm^2/s$ or less.

<32> The method according to any one of <28> to <31>, in which the content of the component (E) in the first agent is preferably 0.1% by mass or more and 30% by mass or less, more preferably 0.3% by mass or more and 25% by mass or less, still more preferably 0.5% by mass or more and 20% by mass or less, even more preferably 1% by mass or more and 15% by mass or less, and even more preferably 1% by mass or more and 10% by mass or less.

<33> The method according to any one of <1> to <32>, in which the first agent further contains a solvent, preferably contains one or more solvents selected from the group consisting of ethanol, 1-propanol, 2-propanol, dimethylpolysiloxane having a viscosity at 25°C of 4 $mm^2/s$ or less, methyltrimethicone, pentane, isopentane, hexane, isohexane, heptane, isoheptane, decane, isodecane, dodecane, isododecane, tridecane, isotridecane, tetradecane, isotetradecane, and light liquid isoparaffin, more preferably contains one or more solvents selected from the group consisting of dimethylpolysiloxane having a viscosity at 25°C of 4 $mm^2/s$ or less, methyltrimethicone, isodecane, isododecane, isotetradecane, and light liquid isoparaffin, and still more preferably contains one or more solvents selected from the group consisting of isodecane, isododecane, isotetradecane, and light liquid isoparaffin.

<34> The method according to <33>, in which the content of the solvent in the first agent is preferably 40% by mass or more and 99.94% by mass or less, more preferably 50% by mass or more and 98% by mass or less, and still more preferably 60% by mass or more and 98% by mass or less.

<35> The method according to any one of <1> to <34>, in which the first agent is a nonaqueous composition, and the content of water is less than 1% by mass, preferably less than 0.5% by mass, and more preferably less than

0.1% by mass.

<36> The method according to any one of <1> to <35>, in which the first agent is a cosmetic composition, preferably a hair cosmetic composition, more preferably a conditioning agent composition, a treatment agent composition, a styling agent composition, or a hair dye composition, and still more preferably a hair dye composition.

<37> The method according to any one of <1> to <36>, in which the first agent is a leave-on preparation.

<38> The method according to any one of <1> to <37>, in which in the step (I), the first agent is applied to the keratin substance or the fiber for a head decoration product in a dry state.

<39> The method according to any one of <1> to <38>, in which the step (I) includes a step of drying after applying the first agent to the keratin substance or the fiber for a head decoration product.

<40> The method according to any one of <1> to <39>, in which the viscosity of the component (C) at 25°C is preferably 300 mPa·s or less, more preferably 200 mPa·s or less, still more preferably 180 mPa·s or less, even more preferably 100 mPa·s or less, even more preferably 50 mPa·s or less, and even more preferably 40 mPa·s or less, and is preferably 1 mPa·s or more.

<41> The method according to any one of <1> to <40>, in which the component (C) is preferably one or more selected from the group consisting of light liquid isoparaffin, isododecane, dimethylpolysiloxane having a viscosity at 25°C of 50 $mm^2/s$ or less, liquid isoparaffin, and triethylhexanoin, and more preferably one or more selected from the group consisting of light liquid isoparaffin, isododecane, dimethylpolysiloxane having a viscosity at 25°C of 50 $mm^2/s$ or less, and triethylhexanoin.

<42> The method according to any one of <1> to <41>, in which the second agent further contains a surfactant as a component (D).

<43> The method according to <42>, in which the component (D) is preferably one or more selected from the group consisting of an anionic surfactant, an amphoteric surfactant, and a nonionic surfactant, more preferably one or more selected from the group consisting of polyoxyethylene alkyl ether sulfate, alkyl hydroxysulfobetaine, and polyoxyethylene sorbitol fatty acid ester, and still more preferably one or more selected from the group consisting of sodium laureth sulfate, lauryl hydroxysulfobetaine, and polyoxyethylene sorbitol mono-, di-, tri-, or tetra-oleic acid.

<44> The method according to any one of <1> to <43>, in which the content of the component (C) in the second agent is preferably 20% by mass or more, more preferably 30% by mass or more, still more preferably 40% by mass or more, even more preferably 50% by mass or more, even more preferably 70% by mass or more, even more preferably 80% by mass or more, and even more preferably 90% by mass or more, and is 100% by mass or less.

<45> The method according to any one of <42> to <44>, in which the content of the component (D) in the second agent is preferably 0.1% by mass or more and 70% by mass or less, more preferably 0.5% by mass or more and 60% by mass or less, still more preferably 1% by mass or more and 50% by mass or less, even more preferably 5% by mass or more and 50% by mass or less, and even more preferably 10% by mass or more and 50% by mass or less.

<46> The method according to any one of <42> to <45>, in which the total content of the component (C) and the component (D) in the second agent is preferably 30% by mass or more, more preferably 50% by mass or more, still more preferably 60% by mass or more, even more preferably 70% by mass or more, even more preferably 80% by mass or more, even more preferably 90% by mass or more, even more preferably 95% by mass or more, and even more preferably 98% by mass or more, and is 100% by mass or less.

<47> The method according to any one of <42> to <46>, in which the mass ratio of the content of the component (D) to the content of the component (C) [(D)/(C)] in the second agent is preferably 0.005 or more and 10 or less, more preferably 0.010 or more and 5 or less, still more preferably 0.010 or more and 3 or less, even more preferably 0.010 or more and 2 or less, even more preferably 0.010 or more and 1.5 or less, and even more preferably 0.010 or more and 1.2 or less.

<48> The method according to any one of <1> to <47>, in which in the step (II), the second agent and a detergent are applied to the keratin substance or the fiber for a head decoration product after the film formation by the step (I).

<49> The method according to <48>, in which the detergent is preferably a detergent containing water as a main component, and more preferably a detergent containing a surfactant and water and containing water as a main component.

<50> The method according to <49>, in which the content of the surfactant in the detergent is preferably 0.01% by mass or more and 50% by mass or less, more preferably 0.1% by mass or more and 40% by mass or less, and still more preferably 1% by mass or more and 30% by mass or less.

<51> The method according to <49> or <50>, in which the content of water in the detergent is preferably 50% by mass or more, more preferably 60% by mass or more, and still more preferably 70% by mass or more, and is preferably 99.99% by mass or less, more preferably 99.9% by mass or less, and still more preferably 99% by mass or less.

<52> The method according to any one of <48> to <51>, in which in the step (II), the detergent is applied to the keratin substance or the fiber for a head decoration product before applying the second agent, at the same time as

applying the second agent, or after applying the second agent, and preferably after applying the second agent.
<53> A kit for treating a keratin substance or a fiber for a head decoration product, including: a first agent containing a component (A): a silicone film-forming agent that is solid at 25°C, and a component (B): an organopolysiloxane other than the component (A), which has a cationic group; and a second agent containing a component (C): an oil agent.

Examples

Hereinafter, the present invention will be described with reference to Examples, but the present invention is not limited to the scope of Examples. In these Examples, various measurements and evaluations were performed by the following methods.

<Viscosity>

The viscosity of the component (C) at 25°C was measured using a TVB-10 viscometer manufactured by Toki Sangyo Co., Ltd. under the following conditions.

(Measurement Conditions)

Rotor: No. 1, rotation speed: 60 rpm, 60 seconds

However, when the measurement result exceeded 180 mPa·s, the rotation speed was changed to 30 rpm.

<Finger Combability of Hair After Step (I)>

A bundle of human gray hair (100%) (manufactured by Beaulax Corporation, length: 10 cm, mass: 1 g) was washed with a plain shampoo having the following composition, rinsed with warm water at 40°C, and sufficiently dried. The first agent 0.2 g described in the tables was applied to the hair bundle with a finger and allowed to spread, and then warm air was blown using a dryer, and the hair bundle was dried for 30 seconds while passing through a comb, and then further dried for 60 seconds without passing through a comb. After drying, the hair bundle was further passed through a comb five times to prepare a hair bundle after the first agent treatment (step (I)).
The finger combability of the hair bundle after the above-described treatment was subjected to sensory evaluation by a specialized panelist according to the following criteria, and the total score of N = 2 was calculated.
When the total score is 3 points or less, it is felt that the finger combability is not good. When the total score is 4 points or more, the finger combability is good, and when the total score is 7 points or more, the finger combability is further good.

4: Feel that the finger combability is good
3: Feel that the finger combability is slightly good
2: Feel that the finger combability is slightly poor
1: Feel that the finger combability is poor

(Composition of Plain Shampoo)

| Components (% by mass) | |
|---|---|
| Sodium polyoxyethylene(2) lauryl ether sulfate (*1) | 15.5 |
| Lauric acid diethanolamide (*2) | 1.5 |
| Edetate tetrasodium salt | 0.3 |
| Sodium benzoate | 1.43 |
| Purified water | balance |
| Total | 100.0 |

*1: 57.4% by mass as EMAL 227 (manufactured by Kao Corporation, active ingredient 27% by mass)
*2: AMINON L-02 (manufactured by Kao Corporation)

<Hair Washing Resistance of Hair After Step (I)>

A bundle of human gray hair (100%) (manufactured by Beaulax Corporation, length: 10 cm, mass: 1 g) was washed with a plain shampoo having the above-described composition, rinsed with warm water at 40°C, and sufficiently dried. The first agent 0.2 g described in the tables was applied to the hair bundle with a finger and allowed to spread, and then warm air was blown using a dryer, and the hair bundle was dried for 30 seconds while passing through a comb, and then further dried for 60 seconds without passing through a comb. Further, after drying for 2 days or more at room temperature, each hair bundle was combed through five times to prepare a hair bundle after the first agent treatment (step (I)).

The treated hair bundle was color-measured with a D65 light source of a color difference meter (manufactured by Konica Minolta, Inc., CR-400) in the CIE color system (L*, a*, b*).

Next, a process of washing the treated hair bundle with warm water at 40°C using a plain shampoo having the composition described above and drying the hair bundle was performed three times. The hair bundle after being washed and dried three times was similarly color-measured by a color difference meter, and the color difference (ΔE*) from the hair bundle after the first agent treatment was calculated according to the following equation. The measurement of L*, a*, and b* was performed at six different points on the hair bundle (one point on each of the front and back sides of the central portion of each region obtained by dividing the hair bundle into three equal parts in the length direction), and the average value was calculated. A smaller value of ΔE* means more excellent hair washing resistance.

$$\Delta E^* = \sqrt{(L_1^* - L_0^*)^2 + (a_1^* - a_0^*)^2 + (b_1^* - b_0^*)^2}$$

$L_0^*$, $a_0^*$, $b_0^*$: measured values of hair bundle after the first agent treatment and before hair washing
$L_1^*$, $a_1^*$, $b_1^*$: measured values of hair bundle after the first agent treatment and after three times of hair washing

<Removability of Film by Step (II)>

A bundle of human gray hair (100%) (manufactured by Beaulax Corporation, length: 10 cm, mass: 1 g) was washed with a plain shampoo having the above-described composition, rinsed with warm water at 40°C, and sufficiently dried. The first agent 0.2 g described in the tables was applied to the hair bundle with a finger and allowed to spread, and then warm air was blown using a dryer, and the hair bundle was dried for 30 seconds while passing through a comb, and then further dried for 60 seconds without passing through a comb. The hair bundle was further dried at room temperature for 2 days or more. Each hair bundle was combed through five times to prepare a hair bundle after the first agent treatment (step (I)).

The treated hair bundle was color-measured with a color difference meter (manufactured by Konica Minolta, Inc., CR-400) in the CIE color system (L*, a*, b*).

Next, 1 g of the second agent described in the tables was applied to the treated hair bundle for 30 seconds with fingers, and then 1 g of the plain shampoo having the above composition was further applied to the hair bundle for 30 seconds with fingers, and the hair bundle was washed away with warm water at 40°C for 15 seconds (step (II)). Next, the hair bundle was sufficiently dried by applying hot air using a dryer. The hair bundle after drying was similarly color-measured by a color difference meter, and the color difference (ΔE*) from the hair bundle before treatment with the second agent was calculated according to the above. A larger value of ΔE* means higher removability of the film formed by the first agent.

<Quick-Drying Property of Hair After Step (II)>

After the steps up to the step (II) were performed in the same manner as in the evaluation of the "Removability of Film by Step (II)", the time required for drying when the hair was dried by applying warm air using a dryer was measured. After drying for 30 seconds while combing, and then for 60 seconds without combing, the hair bundle was touched with a finger at the time points of 90 seconds had elapsed and 210 seconds had elapsed to confirm whether or not the hair bundle was wet.

A: Dried within 90 seconds
B: Dried within 210 seconds
C: Took more than 210 seconds to dry

<Feel of Hair After Step (II)>

In the same manner as in the evaluation of the "Removability of Film by Step (II)", the steps up to drying of the hair bundle after step (II) were carried out. The obtained hair bundle was subjected to sensory evaluation by a specialized panelist according to the following criteria, and the total score of N = 2 was calculated.
When the total score is 3 points or less, it is felt sticky. When the total score is 5 points or more, there is no stickiness, and when it is 6 points or more, there is further no stickiness.

4: Not sticky
3: Not slightly sticky
2: Slightly sticky
1: Sticky

Examples 1 to 11 and Comparative Examples 1 to 3 (Hair Treatment and Evaluation)

The respective components shown in "First agent" of Table 1 were blended at the blending ratios shown in the table, and mixed until homogeneous to prepare a first agent which is a hair dye. Evaluation was performed by the above-described method using the obtained first agent and isododecane as the second agent. The results are shown in Table 1.

Examples 12 to 21 and Comparative Example 4 (Hair Treatment and Evaluation)

The respective components shown in "First Agent" of Table 2 were blended at the blending ratios shown in the table, and mixed until homogeneous to prepare a first agent which is a hair dye. Similarly, the respective components shown in "Second agent" of Table 2 were blended at the blending ratios shown in the table and sufficiently mixed to prepare a second agent. The obtained first agent and second agent were used to perform evaluation by the above-described method. The results are shown in Table 2.
The blending amounts (% by mass) described in the tables are all amounts of active ingredients.

Table 1

| (% by mass) | | | | Example 1 | Example 2 | Example 3 | Example 4 | Example 5 |
|---|---|---|---|---|---|---|---|---|
| First agent | (A) | (A1-2) Trimethylsiloxy silicate | X-21-5595 *1 | 7.5 | 6.6 | 7.5 | 7.5 | 7.5 |
| | | (A2-1) (Acrylates/dimethicone) copolymer | KP-550 *2 | | 0.9 | | | |
| | (A') | (A') Aminopropyltriethoxysilane | Aminopropyltriethoxysilane (98%) *3 | | | | | |
| | (B) | (B2-2) PEG-amodimethicone | SS-3588 *4 | 1.5 | 1.5 | | | |
| | | (B1) Aminoethylaminopropyl dimethicone | KF-8005S *5 | | | 1.5 | | |
| | | (B1)Aminopropyl dimethicone | KF-8015 *6 | | | | 1.5 | |
| | | (B1) Amodimethicone | XF42-B1989 *7 | | | | | 1.5 |
| | | (B1)Bis-cetearyl amodimethicone | SilsoftAX *8 | | | | | |
| | (B') | (VA/crotonic acid/vinyl neodecanoate) copolymer | RESYN 28-2930 *9 | | | | | |
| | Colorant | Carbon black | ISD-CB2 *10 | 1.5 | 1.5 | 1.5 | 1.5 | 1.5 |
| | Volatile solvent | Isododecane | Marukazol R *11 | Balance | Balance | Balance | Balance | Balance |
| | | Ethanol | 99-degree Synthetic alcohol *12 | | | | | |
| | Total | | | 100 | 100 | 100 | 100 | 100 |
| | Component (A) (or Component (A')) content (% by mass) | | | 7.5 | 7.5 | 7.5 | 7.5 | 7.5 |
| | Component (B) content (% by mass) | | | 1.5 | 1.5 | 1.5 | 1.5 | 1.5 |
| | Components (A), (A'), (B) total content (% by mass) | | | 9.0 | 9.0 | 9.0 | 9.0 | 9.0 |
| | (B)/(A) (or (B)/(A')) | | | 0.20 | 0.20 | 0.20 | 0.20 | 0.20 |
| Second agent | (C) | Isododecane (2.9 mPa·s) | Marukazol R *11 | 100 | 100 | 100 | 100 | 100 |

(continued)

| (% by mass) | | | Example | | | | |
|---|---|---|---|---|---|---|---|
| | | | 1 | 2 | 3 | 4 | 5 |
| Evaluation result | Finger combability of hair after step (1) | Finger combability after first agent treatment | 7 | 6 | 5 | 4 | 5 |
| | Hair washing resistance of hair after step (1) | ΔE * of after first agent treatment and after three times of hair washing | 2.2 | 14.7 | 13.2 | 10.5 | 2.9 |
| | Removability of film by step (II) | ΔE * of after removing first agent | 43.3 | 32.7 | 35.8 | 39.0 | 40.2 |
| | Quick-drying property of hair after step (II) | Drying time of hair required after first agent removal treatment | A | A | A | A | A |
| | Feel of hair after step (II) | Stickiness of hair bundle after removal of first agent | 8 | 6 | 8 | 6 | 7 |

Table 1 (continued)

| (% by mass) | | | | Example | | | |
|---|---|---|---|---|---|---|---|
| | | | | 6 | 7 | 8 | 9 |
| First agent | (A) | (A1-2) Trimethylsiloxy silicate | X-21-5595 *1 | 7.5 | 1.5 | 4.5 | 6 |
| | | (A2-1) (Acrylates/dimethicone) copolymer | KP-550 *2 | | | | |
| | (A') | (A') Aminopropyltriethoxysilane | Aminopropyltriethoxysilane (98%) *3 | | | | |
| | (B) | (B2-2) PEG-amodimethicone | SS-3588 *4 | | 7.5 | 4.5 | 3 |
| | | (B1) Aminoethylaminopropyl dimethicone | KF-8005S *5 | | | | |
| | | (B1)Aminopropyl dimethicone | KF-8015 *6 | | | | |
| | | (B1) Amodimethicone | XF42-B1989 *7 | | | | |
| | | (B1)Bis-cetearyl amodimethicone | SilsoftAX *8 | 1.5 | | | |
| | (B') | (VA/crotonic acid/vinyl neodecanoate) copolymer | RESYN 28-2930 *9 | | | | |
| | Colorant | Carbon black | ISD-CB2 *10 | 1.5 | 1.5 | 1.5 | 1.5 |
| | Volatile solvent | Isododecane | Marukazol R *11 | Balance | Balance | Balance | Balance |
| | | Ethanol | 99-degree Synthetic alcohol *12 | | | | |
| | Total | | | 100 | 100 | 100 | 100 |
| | Component (A) (or Component (A')) content (% by mass) | | | 7.5 | 1.5 | 4.5 | 6 |
| | Component (B) content (% by mass) | | | 1.5 | 7.5 | 4.5 | 3 |
| | Components (A), (A'), (B) total content (% by mass) | | | 9.0 | 9.0 | 9.0 | 9.0 |
| | (B)/(A) (or (B)/(A')) | | | 0.20 | 5.0 | 1.0 | 0.50 |
| Second agent | (C) | Isododecane (2.9 mPa·s) | Marukazol R *11 | 100 | 100 | 100 | 100 |

(continued)

| (% by mass) | | | Example | | | |
|---|---|---|---|---|---|---|
| | | | 6 | 7 | 8 | 9 |
| Evaluation result | Finger combability of hair after step (1) | Finger combability after first agent treatment | 6 | 8 | 8 | 8 |
| | Hair washing resistance of hair after step (1) | $\Delta E$ * of after first agent treatment and after three times of hair washing | 8.6 | 5.8 | 1.7 | 8.4 |
| | Removability of film by step (II) | $\Delta E$ * of after removing first agent | 47.7 | 26.2 | 34.3 | 45.7 |
| | Quick-drying property of hair after step (II) | Drying time of hair required after first agent removal treatment | A | B | B | A |
| | Feel of hair after step (II) | Stickiness of hair bundle after removal of first agent | 8 | 5 | 6 | 8 |

Table 1 (continued)

| (% by mass) | | | | Example | | Comparative Example | | |
|---|---|---|---|---|---|---|---|---|
| | | | | 10 | 11 | 1 | 2 | 3 |
| First agent | (A) | (A1-2) Trimethylsiloxy silicate | X-21-5595 *1 | 8.18 | 6.41 | | | |
| | | (A2-1) (Acrylates/dimethicone) copolymer | KP-550 *2 | | 2.5 | | | |
| | (A') | (A') Aminopropyltriethoxysilane | Aminopropyltriethoxysilane (98%) *3 | | | | | 7.5 |
| | (B) | (B2-2) PEG-amodimethicone | SS-3588 *4 | 0.82 | 0.09 | | | 1.5 |
| | | (B1) Aminoethylaminopropyl dimethicone | KF-8005S *5 | | | | | |
| | | (B1) Aminopropyl dimethicone | KF-8015 *6 | | | | 9 | |
| | | (B1) Amodimethicone | XF42-B1989 *7 | | | | | |
| | | (B1)Bis-cetearyl amodimethicone | SilsoftAX *8 | | | | | |
| | (B') | (VA/crotonic acid/vinyl neodecanoate) copolymer | RESYN 28-2930 *9 | | | 9 | | |
| | Colorant | Carbon black | ISD-CB2 *10 | 1.5 | 1.5 | 1.5 | 1.5 | 1.5 |
| | Volatile solvent | Isododecane | Marukazol R *11 | Balance | Balance | | Balance | Balance |
| | | Ethanol | 99-degree Synthetic alcohol *12 | | | Balance | | |
| | Total | | | 100 | 100 | 100 | 100 | 100 |
| | Component (A) (or Component (A')) content (% by mass) | | | 8.18 | 8.91 | 0 | 0 | 7.5 |
| | Component (B) content (% by mass) | | | 0.82 | 0.09 | 0 | 9 | 1.5 |
| | Components (A), (A'), (B) total content (% by mass) | | | 9.0 | 9.0 | - | 9.0 | 9.0 |
| | (B)/(A) (or (B)/(A')) | | | 0.10 | 0.010 | - | - | 0.20 |
| Second agent | (C) | Isododecane (2.9 mPa·s) | Marukazol R *11 | 100 | 100 | 100 | 100 | 100 |

(continued)

| (% by mass) | | | Example | | Comparative Example | | |
|---|---|---|---|---|---|---|---|
| | | | 10 | 11 | 1 | 2 | 3 |
| Evaluation result | Finger combability of hair after step (1) | Finger combability after first agent treatment | 6 | 7 | 3 | 8 | 6 |
| | Hair washing resistance of hair after step (1) | $\Delta E$ * of after first agent treatment and after three times of hair washing | 6.8 | 10.1 | 46.1 | 16.8 | 35.6 |
| | Removability of film by step (II) | $\Delta E$ * of after removing first agent | 45.8 | 41.6 | | 40.1 | |
| | Quick-drying property of hair after step (II) | Drying time of hair required after first agent removal treatment | A | A | | C | |
| | Feel of hair after step (II) | Stickiness of hair bundle after removal of first agent | 7 | 7 | | 8 | |

Table 2

| | | | | Example | | | |
|---|---|---|---|---|---|---|---|
| | | | | 1 | 12 | 13 | 14 |
| First agent | (A) | (A1-2) Trimethylsiloxy silicate | X-21-5595 *1 | 7.5 | 7.5 | 7.5 | 7.5 |
| | (B) | (B2-2) PEG-amodimethicone | SS-3588 *4 | 1.5 | 1.5 | 1.5 | 1.5 |
| | Colorant | Carbon black | ISD-CB2 *10 | 1.5 | 1.5 | 1.5 | 1.5 |
| | Volatile solvent | Isododecane | Marukazol R *11 | Balance | Balance | Balance | Balance |
| | Total | | | 100 | 100 | 100 | 100 |
| | (B)/(A) | | | 0.20 | 0.20 | 0.20 | 0.20 |
| Second agent | (C) | Isododecane (2.9 mPa·s) | Marukazol R *11 | 100 | | | |
| | | Light liquid paraffin (3.0 mPa·s) | PARLEAM 3 *13 | | 100 | | |
| | | Dimethicone (6.9 mPa·s) | KF-96A-6cs *14 | | | 100 | |
| | | Dimethicone (29.0 mPa·s) | KF-96A-20cs *15 | | | | 100 |
| | | Triethylhexanoin (38.8 mPa·s) | EXCEPARL TGO *16 | | | | |
| | | Liquid paraffin (181.4 mPa·s) | Hycol K-350 *17 | | | | |
| | (D) | Ammonium laureth sulfate | EMAL 125A *18 | | | | |
| | | Lauryl hydroxysulfobetaine | AMPHITOL 20HD *19 | | | | |
| | | Sorbes-30 tetraoleate | RHEODOL 430V *20 | | | | |
| | Total | | | 100 | 100 | 100 | 100 |
| | (D)/(C) | | | 0 | 0 | 0 | 0 |
| Evaluation result | Removability of film by step (II) | | ΔE* of after removing first agent | 43.3 | 49.1 | 43.9 | 39.9 |
| | Quick-drying property of hair after step (II) | | Drying time required after first agent removal treatment | A | A | A | A |
| | Feel of hair after step (II) | | Stickiness of hair bundle after removal of first agent | 8 | 8 | 8 | 8 |

Table 2 (continued)

| | | | | Example | | | |
|---|---|---|---|---|---|---|---|
| | | | | 15 | 16 | 17 | 18 |
| First agent | (A) | (A1-2) Trimethylsiloxy silicate | X-21-5595 *1 | 7.5 | 7.5 | 7.5 | 7.5 |
| | (B) | (B2-2) PEG-amodimethicone | SS-3588 *4 | 1.5 | 1.5 | 1.5 | 1.5 |
| | Colorant | Carbon black | ISD-CB2 *10 | 1.5 | 1.5 | 1.5 | 1.5 |
| | Volatile solvent | Isododecane | Marukazol R *11 | Balance | Balance | Balance | Balance |
| | Total | | | 100 | 100 | 100 | 100 |
| | (B)/(A) | | | 0.20 | 0.20 | 0.20 | 0.20 |
| Second agent | (C) | Isododecane (2.9 mPa·s) | Marukazol R *11 | | | Balance | Balance |
| | | Light liquid paraffin (3.0 mPa·s) | PARLEAM 3 *13 | | | | |
| | | Dimethicone (6.9 mPa·s) | KF-96A-6cs *14 | | | | |
| | | Dimethicone (29.0 mPa·s) | KF-96A-20cs *15 | | | | |
| | | Triethylhexanoin (38.8 mPa·s) | EXCEPARL TGO *16 | 100 | | | |
| | | Liquid paraffin (181.4 mPa·s) | HycolK-350 *17 | | 100 | | |
| | (D) | Ammonium laureth sulfate | EMAL 125A *18 | | | 1 | |
| | | Lauryl hydroxysulfobetaine | AMPHITOL 20HD *19 | | | | 1 |
| | | Sorbes-30 tetraoleate | RHEODOL 430V *20 | | | | |
| | Total | | | 100 | 100 | 100 | 100 |
| | (D)/(C) | | | 0 | 0 | 0.01 | 0.01 |
| Evaluation result | Removability of film by step (II) | | ΔE* of after removing first agent | 48.2 | 10.3 | 47.9 | 46.9 |
| | Quick-drying property of hair after step (II) | | Drying time required after first agent removal treatment | A | B | A | A |
| | Feel of hair after step (II) | | Stickiness of hair bundle after removal of first agent | 8 | 5 | 8 | 7 |

Table 2 (continued)

| | | | | Example | | | Comparative Example |
|---|---|---|---|---|---|---|---|
| | | | | 19 | 20 | 21 | 4 |
| First agent | (A) | (A1-2) Trimethylsiloxy silicate | X-21-5595 *1 | 7.5 | 7.5 | 7.5 | 7.5 |
| | (B) | (B2-2) PEG-amodimethicone | SS-3588 *4 | 1.5 | 1.5 | 1.5 | 1.5 |
| | Colorant | Carbon black | ISD-CB2 *10 | 1.5 | 1.5 | 1.5 | 1.5 |
| | Volatile solvent | Isododecane | Marukazol R *11 | Balance | Balance | Balance | Balance |
| | Total | | | 100 | 100 | 100 | 100 |
| | (B)/(A) | | | 0.20 | 0.20 | 0.20 | 0.20 |
| Second agent | (C) | Isododecane (2.9 mPa·s) | Marukazol R *11 | 99 | 50 | 35 | |
| | | Light liquid paraffin (3.0 mPa·s) | PARLEAM 3 *13 | | | | |
| | | Dimethicone (6.9 mPa·s) | KF-96A-6cs *14 | | | | |
| | | Dimethicone (29.0 mPa·s) | KF-96A-20cs *15 | | | | |
| | | Triethylhexanoin (38.8 mPa·s) | EXCEPARL TGO *16 | | | | |
| | | Liquid paraffin (181.4 mPa·s) | Hycol K-350 *17 | | | | |
| | (D) | Ammonium laureth sulfate | EMAL 125A *18 | | | | |
| | | Lauryl hydroxysulfobetaine | AMPHITOL 20HD *19 | | | | |
| | | Sorbes-30 tetraoleate | RHEODOL 430V *20 | 1 | 50 | 65 | 100 |
| | Total | | | 100 | 100 | 100 | 100 |
| | (D)/(C) | | | 0.01 | 1.00 | 1.86 | - |

(continued)

| | | | Example | | | Comparative Example |
|---|---|---|---|---|---|---|
| | | | 19 | 20 | 21 | 4 |
| Evaluation result | Removability of film by step (II) | $\Delta E^*$ of after removing first agent | 44.1 | 49.1 | 43.4 | 6.8 |
| | Quick-drying property of hair after step (II) | Drying time required after first agent removal treatment | A | A | A | C |
| | Feel of hair after step (II) | Stickiness of hair bundle after removal of first agent | 7 | 8 | 7 | 3 |

The ingredients shown in the tables are as follows.

*1: X-21-5595, manufactured by Shin-Etsu Chemical Co., Ltd., isododecane solution of trimethylsiloxysilicate (60% by mass)
*2: KP-550, manufactured by Shin-Etsu Chemical Co., Ltd., isododecane solution of (acrylates/dimethicone) copolymer (40% by mass)
*3: Aminopropyltriethoxysilane, manufactured by FUJIFILM Wako Pure Chemical Corporation, Aminopropyltriethoxysilane (98%)
*4: DOWSII, SS-3588 Fluid, manufactured by Dow Toray Co., Ltd., ethanol solution of (bisisobutyl PEG-15/amodimethicone) copolymer (80% by mass)
*5: KF-8005S, manufactured by Shin-Etsu Chemical Co., Ltd., aminoethylaminopropyl dimethicone
*6: KF-8015, manufactured by Shin-Etsu Chemical Co., Ltd., aminopropyl dimethicone
*7: XF42-B1989, manufactured by Momentive Performance Materials Japan LLC, amodimethicone (aminoethylaminopropylmethylsiloxane-dimethylsiloxane copolymer)
*8: Silsoft AX, manufactured by Momentive Performance Materials Japan LLC, bis-cetearyl amodimethicone
*9: RESYN 28-2930, manufactured by Nouryon Japan K.K., (VA/crotonic acid/vinyl neodecanoate) copolymer
*10: ISD-CB2, manufactured by Daito Kasei Kogyo Co., Ltd., isododecane solution of carbon black (15% by mass)
*11: Marukazol R, manufactured by Maruzen Petrochemical Co., Ltd., isododecane
*12: 99-degree synthetic alcohol, manufactured by Japan Synthetic Alcohol Co., Ltd., ethanol
*13: PARLEAM 3, manufactured by NOF CORPORATION, hydrogenated polyisobutene
*14: KF-96A-6cs, manufactured by Shin-Etsu Chemical Co., Ltd., dimethicone
*15: KF-96A-20cs, manufactured by Shin-Etsu Chemical Co., Ltd., dimethicone
*16: EXCEPARL TGO, manufactured by Kao Corporation, triethylhexanoin
*17: Hycol K-350, manufactured by Kao Corporation, liquid paraffin
*18: EMAL 125A, manufactured by Kao Corporation, aqueous solution of ammonium laureth sulfate (25% by mass)
*19: AMPHITOL 20HD, manufactured by Kao Corporation, aqueous solution of lauryl hydroxysulfobetaine (30% by mass)
*20: RHEODOL 430V, manufactured by Kao Corporation, Sorbes-30 tetraoleate

From Table 1, according to the treatment method of the present invention, the durability of the hair treatment effect (hair washing resistance) by the first agent in the step (I) and the feel of the hair after the treatment are good. In addition, in the step (II), the film formed by using the first agent in the step (I) can be easily removed by the second agent, and further, the quick-drying property and the feel of the hair after the film removal in the step (II) are both excellent.
Comparative Example 1 is an example in which a hair dye corresponding to the invention of PTL 1 was used as the first agent, but at the time of the treatment with the first agent in the step (I), both the durability of the hair treatment effect (hair washing resistance) and the feel of the hair after the treatment were poor. Comparative Example 2 is an example in which the first agent not containing the component (A) was used, and the durability of the hair treatment effect by the first agent in the step (I) and the quick-drying property of the hair after the film removal in the step (II) were deteriorated. Comparative Example 3 is an example using a first agent in which a silicone compound that is liquid at 25°C (described as component (A') in the table), which does not correspond to the component (A), was blended instead of the component (A), and the result was that the durability of the hair treatment effect (hair washing resistance) was inferior at the time of the treatment with the first agent in the step (I).
Table 2 shows examples in which the composition of the second agent used in the step (II) was changed. It is found that when the viscosity at 25°C of the component (C) is low (in particular, 180 mPa·s or less), the effect of removing the film by the step (II) is high. In addition, from the comparison between Example 1 and Examples 17 to 21, when the component (C) and the component (D) are used in the second agent, the effect of removing the film by the step (II) is improved.
However, as shown in Comparative Example 4, it is found that when the second agent does not contain the component (C), both the effect of removing the film by the step (II) and the quick-drying property and the feel of the hair after film removal in the step (II) are deteriorated.

Industrial Applicability

According to the present invention, it is possible to provide a treatment method in which the durability of the treatment effect of the keratin substance or the fiber for a head decoration product by the treatment agent is high, the feel after the treatment is good, the treatment agent can be easily removed from the keratin substance or the fiber for a head decoration product, and both the quick-drying property and the feel of the keratin substance or the fiber for a head decoration product after removal of the treatment agent are excellent. For example, when the first agent is a hair

dye containing a colorant, the hair dyed with the hair dye has high hair washing resistance, excellent color durability, and good feel after dyeing. In addition, the coloring by the hair dye can be easily restored by treatment with the second agent, and the hair after removal of the coloring is excellent in quick-drying property and feel.

## Claims

1. A method for treating a keratin substance or a fiber for a head decoration product, the method comprising, in the following order,

   a step (I) of applying a first agent to the keratin substance or the fiber for a head decoration product to form a film, and
   a step (II) of applying a second agent to the keratin substance or the fiber for a head decoration product on which the film has been formed to remove the film,
   wherein the first agent contains a component (A): a silicone film-forming agent that is solid at 25°C, and a component (B): an organopolysiloxane other than the component (A), which has a cationic group, and the second agent contains a component (C): an oil agent.

2. The method according to claim 1, wherein in the step (II), the second agent and a detergent are applied to the keratin substance or the fiber for a head decoration product after the film formation.

3. The method according to claim 1 or 2, wherein the component (A) is one or more selected from the group consisting of the following components (A1) and (A2):

   component (A1): a silicone resin represented by an average formula $(R^1)_mSiO_{(4-m)/2}$ (wherein, $R^1$ represents a hydrocarbon group having 1 or more and 12 or less carbon atoms which may be fluorine-substituted or a hydroxy group; a plurality of $R^1$'s may be the same as or different from each other; and m is an average number which is more than 0 and less than 3), the silicone resin containing one or more units selected from the group consisting of a T unit represented by $R^1SiO_{3/2}$ and a Q unit represented by $SiO_{4/2}$; and
   component (A2): a silicone polymer containing a polysiloxane moiety and a moiety formed of a non-silicone organic chain.

4. The method according to any one of claims 1 to 3, wherein the component (B) is one or more selected from the group consisting of an amino-modified silicone (B 1) and an aminopolyether-modified silicone (B2).

5. The method according to any one of claims 1 to 4, wherein a ratio [(B)/(A)] of a content mass of the component (B) to the component (A) in the first agent is 0.010 or more and 50 or less.

6. The method according to any one of claims 1 to 5, wherein the component (C) has a viscosity at 25°C of 300 mPa·s or less.

7. The method according to any one of claims 1 to 6, wherein the first agent further contains a colorant.

8. The method according to any one of claims 1 to 7, wherein the second agent further contains a surfactant as a component (D).

9. The method according to any one of claims 1 to 8, wherein the keratin substance is hair.

10. A kit for treating a keratin substance or a fiber for a head decoration product, comprising: a first agent containing a component (A): a silicone film-forming agent that is solid at 25°C, and a component (B): an organopolysiloxane other than the component (A), which has a cationic group; and a second agent containing a component (C): an oil agent.

**INTERNATIONAL SEARCH REPORT**

International application No.
**PCT/JP2022/043778**

**A. CLASSIFICATION OF SUBJECT MATTER**

***A61K 8/89***(2006.01)i; ***A61K 8/894***(2006.01)i; ***A61Q 3/02***(2006.01)i; ***A61Q 5/10***(2006.01)i
FI: A61K8/89; A61K8/894; A61Q3/02; A61Q5/10

According to International Patent Classification (IPC) or to both national classification and IPC

**B. FIELDS SEARCHED**

Minimum documentation searched (classification system followed by classification symbols)

A61K8/89; A61K8/894; A61Q3/02; A61Q5/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | US 2009/0074702 A1 (L'OREAL S. A.) 19 March 2009 (2009-03-19)<br>claims, paragraphs [0001], [0008]-[0010], [0141]-[0168], [0354], [0358], [0359], [0363] | 1-10 |
| Y | JP 2021-123536 A (SHISEIDO CO,, LTD.) 30 August 2021 (2021-08-30)<br>claims, paragraphs [0016]-[0019], [0050], [0055] | 1-10 |
| A | JP 2013-541585 A (AVON PRODUCTS, INC.) 14 November 2013 (2013-11-14)<br>entire text | 1-10 |
| A | JP 2011-519853 A (AVON PRODUCTS, INC.) 14 July 2011 (2011-07-14)<br>entire text | 1-10 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 January 2023** | **31 January 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2022/043778**

| Patent document cited in search report | Publication date (day/month/year) | Patent family member(s) | Publication date (day/month/year) |
|---|---|---|---|
| US 2009/0074702 A1 | 19 March 2009 | (Family: none) | |
| JP 2021-123536 A | 30 August 2021 | WO 2021/153624 A1 | |
| JP 2013-541585 A | 14 November 2013 | US 2012/0110752 A1<br>entire text<br>WO 2012/061025 A1<br>EP 2635349 A1 | |
| JP 2011-519853 A | 14 July 2011 | US 2009/0274640 A1<br>entire text<br>EP 2280683 A1<br>WO 2009/134555 A2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2006063059 A
- JP H111530 A
- JP 2000063225 A
- JP H225411 A
- JP H2132141 A
- JP H3162442 A
- JP 2003104825 A
- JP H692825 A
- JP 2003342139 A

### Non-patent literature cited in the description

- **NAKAMUTA.** *Nihon Kagakukai-shi,* 1956, 77588